# EUROPEAN PATENT SPECIFICATION

(11) **EP 2 403 877 B1**
(45) Date of publication and mention of the grant of the patent: **06.05.2015**
(21) Application number: 10706164.0
(22) Date of filing: 01.03.2010
(51) Int. Cl.: C07K 16/28, G01N 33/574, A61P 35/00

(54) **PHARMACEUTICAL COMPOSITION AND METHOD FOR IDENTIFYING A CANCEROUS AND/OR AN INFLAMMATORY DISEASE IN A PATIENT**
PHARMAZEUTISCHE ZUSAMMENSETZUNG UND VERFAHREN ZUR IDENTIFIZIERUNG EINER KREBSERKRANKUNG UND/ODER ENTZÜNDLICHEN ERKRANKUNG IN EINEM PATIENTEN
COMPOSITION PHARMACEUTIQUE ET PROCÉDÉ D'IDENTIFICATION D'UNE MALADIE CANCÉREUSE ET/OU INFLAMMATOIRE CHEZ UN PATIENT

(30) Priority: 06.03.2009 US 158083 P
(43) Date of publication of application: 11.01.2012
(73) Proprietor: Klaus Tschira Stiftung gGmbH, 69118 Heidelberg (DE)
(72) Inventor: SCHUBERT, Walter, 39175 Biederitz (DE)
(74) Representative: Hofstetter, Schurack & Partner
(86) International application number: PCT/EP2010/001254
(87) International publication number: WO 2010/099918

(56) References cited:
- EP-A1- 1 083 226
- EP-B1- 1 181 525
- WO-A1-02/092127
- WO-A1-2008/114876
- WO-A2-2008/008284
- WO-A2-2009/009114
- US-A1- 2006 019 235
- THOMPSON MICHAEL A ET AL: "CD26/dipeptidyl peptidase IV as a novel therapeutic target for cancer and immune disorders" MINI REVIEWS IN MEDICINAL CHEMISTRY, BENTHAM SCIENCE PUBLISHERS, HILVERSUM, NL, vol. 7, no. 3, 1 March 2007 (2007-03-01), pages 253-273, XP009133764 ISSN: 1389-5575
- HAVRE PAMELA A ET AL: "The role of CD26/dipeptidyl peptidase IV in cancer." FRONTIERS IN BIOSCIENCE : A JOURNAL AND VIRTUAL LIBRARY 2008 LNKD- PUBMED:17981655, vol. 13, 2008, pages 1634-1645, XP002542251 ISSN: 1093-4715
- INAMOTO T ET AL: "Anti-CD26 monoclonal antibody-mediated G1-S arrest of human renal clear cell carcinoma Caki-2 is associated with retinoblastoma substrate dephosphorylation, cyclin-dependent kinase 2 reduction, p27(kip1) enhancement, and disruption of binding to the" CLINICAL CANCER RESEARCH, THE AMERICAN ASSOCIATION FOR CANCER RESEARCH, US LNKD- DOI:10.1158/1078-0432.CCR-06-0361, vol. 12, no. 11, PART 1, 1 January 2006 (2006-01-01), pages 3470-3477, XP008116675 ISSN: 1078-0432
- PARK CATHERINE C ET AL: "Beta1 integrin inhibitory antibody induces apoptosis of breast cancer cells, inhibits growth, and distinguishes malignant from normal phenotype in three dimensional cultures and in vivo." CANCER RESEARCH 1 FEB 2006 LNKD- PUBMED:16452209, vol. 66, no. 3, 1 February 2006 (2006-02-01), pages 1526-1535, XP007913210 ISSN: 0008-5472
- MASUMOTO A ET AL: "Role of beta1 integrins in adhesion and invasion of hepatocellular carcinoma cells." HEPATOLOGY (BALTIMORE, MD.) JAN 1999 LNKD- PUBMED:9862852, vol. 29, no. 1, January 1999 (1999-01), pages 68-74, XP007913209 ISSN: 0270-9139

## Description

### FIELD OF THE INVENTION

The invention relates to a method for identifying prostate cancer in a patient as defined in the appendant claims.

### BACKGROUND OF THE INVENTION

Among the non-cutaneous malignant neoplasm prostate cancer is the most common one in men in western countries. The disease accounts for the deaths of approx. 30,000 men per year in the USA. The pathogenesis of prostate cancer is unclear, although it was suggested that chronic inflammation is involved in the carcinogenesis. In the drive to find altered molecular pathways in the process of prostatic carcinogenesis, proteomics techniques have been applied to identify multiple changes in protein abundances simultaneously. In a recent study hundreds of proteins were found to be up-regulated in prostate cancer (TNM stages T1-T3), and dozens were found to be downregulated. Many of these proteins were suggested to account for aberrant behaviour of multiple signalling pathways in prostate cancer. An important question remains to be answered: Where are specific pathways and their networks located *in situ* and how are they structured? A proteomic technology addressing these features directly must be (i) non-destructive, (ii) able to visualize a large number of different molecular cell components (MCC) (preferentially proteins) simultaneously, and (iii) alignable with histological structures that can be readily examined in routine diagnostics working on light microscopic scales. The same considerations also apply to other cancerous and inflammatory diseases.

Document WO 02/092127 A1 discloses therapeutic methods comprising administering anti-CD26 antibodies for the prevention and treatment of cancers and immune diseases associated with expressing CD26. Various types of anti-CD26 antibodies and modes of administration are described.

Document WO 2008/114876 A1 discloses a therapeutic agent for malignant mesothelioma comprising a substance which inhibits binding of CD26 to extracellular matrix such as an siRNA targeting CD26 cDNA or an anti-CD26 antibody.

From the WO 2009/009114 A2 compositions and methods for characterizing, diagnosing, and treating cancer are known. In particular, document WO 2009/009114 A2 identifies integrin beta (1) as a cancer stem cell marker.

It is an object of the invention to ameliorate the identification of prostate cancer.

### SUMMARY OF THE INVENTION

The foregoing object is inventively achieved through a method according to claim 1 for identifying prostate cancer in a patient,

Advantageous embodiments with expedient further developments of the invention are indicated in the corresponding subclaims.

A method for treating a cancerous disease in a patient comprises internally administering to the patient an effective amount of a pharmaceutical preparation blocking the biological activity of at least one protein cluster (CMP) or group of CMPs containing at least CD26 and/or CD29. CD26 and CD29 in this context are cell surface molecules that can be identified via the so-called "cluster of differentiation" or "cluster of designation" (abbreviated as CD) protocol. A protein cluster within the scope of the present disclosure is to be taken as combinatorial molecular phenotypes (CMPs) describing colocated and/or anti-colocated CDs, proteins and/or molecules in a certain place of a cell. Accordingly, groups of CMPs represent regions of colocated and/or anti-colocated CDs, proteins and/or molecules in a cell. The CMPs in this connection may for instance be indicated in the form of a binary code, with it being possible to encode via any digit of the binary number (i.e. via any "bit") whether a predetermined protein/molecule is given in a certain place of the cell or in a concentration exceeding a certain limit value (1 or True), or else is not given in this place of the cell or only in a concentration falling short of a predetermined limit value (0 or False). The invention is based on the insight that CD26 and CD29 within the context of prostate cancer are so-called "lead proteins". On a high level of molecular cell organization, protein clusters are frequently interlocked as protein cluster networks (corresponding to said CMP motifs), which are controlled by said lead proteins: when the lead protein is inhibited, the clusters disassemble leading to loss of function. Therefore the cancerous disease can effectively and without side-effects be cured by administering to the patient an effective amount of said pharmaceutical preparation blocking the biological activity of at least one protein cluster (CMP) or group of CMPs containing at least CD26 and/or CD29. In its most simple embodiment the pharmaceutical preparation used in the method can be designed to block the biological activity of a CMP consisting of CD26 and/or CD29 only. A protein cluster or protein group (CMP) blocked by means of the disclosed method besides CD26 and/or CD29 may in principle also comprise further CDs, proteins and/or molecules, for example one or more CDs and molecules as listed in the Supplementary Table (see Annex).

The patient may be a human and/or an animal. In this context it has proven to be advantageous if said pharmaceutical preparation is designed to be type and/or species-specific.

If the disease is prostate cancer, the treatment of the patient with the pharmaceutical preparation - which is blocking the biological activity of the at least CD26 and/or CD29 comprising CMPs - has turned out to be particularly effective.

The pharmaceutical preparation may be administered if a protein cluster (CMP) or a group of CMPs also containing CD44 and/or CD54 and/or CD138 and lacking at least one of CD3, CD4, CD8, CD10, CD13, CD19, CD20, CD38, CD49d, CD58, and CD80 is identified on cell surfaces of cells obtained from an affected part of the body of the patient. In this way a particularly effective treatment of the cancerous disease is ensured, so that in most cases it is sufficient to treat the patient with a low dose of the pharmaceutical preparation. The protein cluster or protein group (CMP) in principle may also lack several or all CDs form the group of CD3, CD4, CD8, CD10, CD13, CD19, CD20, CD38, CD49d, CD58, and CD80.

Said cells may be obtained from a tissue block and/or a single tissue section, the tissue being removed from the prostate of the patient. This allows for a particularly simple and reliable test for the presence of the previously described CMPs and for the presence of prostrate cancer.

The disclosure in a first aspect relates to a use of a pharmaceutical preparation blocking the biological activity of at least one protein cluster (CMP) or group of CMPs containing at least CD26 and/or CD29 for the preparation of a medicament for the treatment of a cancerous disease in a patient. The cancerous disease to be treated is prostate cancer. In this case the pharmaceutical preparation - which is blocking the biological activity of the at least CD26 and/or CD29 comprising CMPs - has proven to be particularly effective. Further, said pharmaceutical preparation comprises a protein cross-linking agent. This facilitates a particularly specific blocking of the biological activity of the at least one protein cluster (CMP) or group of CMPs. Moreover, the pharmaceutical preparation is also low in side-effects. The disclosure is based on the insight that CD26 and/or CD29 within the context of cancerous diseases are so-called "lead proteins". On a high level of molecular cell organization, protein clusters are frequently interlocked as protein cluster networks (corresponding to said CMP motifs), which are controlled by said lead proteins: when the lead protein is inhibited, the clusters disassemble leading to loss of function. Therefore the cancerous disease can be effectively and without side-effects be cured by a medicament comprising said pharmaceutical preparation. In its most simple embodiment the medicament may be designed to block the biological activity of a CMP consisting of CD26 and/or CD29 only. A protein cluster or protein group (CMP) to be blocked by means of the medicament besides CD26 and/or CD29 in principle may also comprise further CDs, proteins and/or molecules, for example one or more CDs and/or molecules as listed in the Supplementary Table (see Annex).

Another embodiment of the disclosure provides a pharmaceutical composition for the treatment of a cancerous disease in a patient comprising a pharmaceutical preparation blocking the biological activity of at least one protein cluster (CMP) or group of CMPs containing at least CD26 and/or CD29. The cancerous disease to be treated is prostate cancer. In this case the pharmaceutical preparation - which is blocking the biological activity of the at least CD26 and/or CD29 comprising CMPs - has proven to be particularly effective. Further, said pharmaceutical preparation comprises a protein cross-linking agent. This facilitates a particularly specific blocking of the biological activity of the at least one protein cluster (CMP) or group of CMPs. Moreover, the pharmaceutical preparation is also low in side-effects. The disclosure in this regard is based on the insight that CD26 and/or CD29 within the context of cancerous diseases are so-called "lead proteins". On a high level of molecular cell organization, protein clusters are frequently interlocked as protein cluster networks (corresponding to said CMP motifs), which are controlled by said lead proteins: when the lead protein is inhibited, the clusters disassemble leading to loss of function. Therefore the cancerous disease can effectively and without side-effects be cured by use of said pharmaceutical preparation. In its most simple embodiment the pharmaceutical preparation may be designed to block the biological activity of a CMP consisting of CD26 and/or CD29 only. A protein cluster or protein group (CMP) to be blocked by means of the pharmaceutical preparation may besides CD26 and/or CD29 in principle also contain further CDs, proteins and/or molecules, for example one or more CDs and/or molecules as listed in the Supplementary Table (see Annex).

In a further aspect of the disclosure said pharmaceutical preparation comprises an antibody, preferably a monoclonal antibody, and/or at least one of TSPAN4, CD9, Filamin, FLNB, CD81, CD46, MAP4K4, FHL2, NME1, PKC alpha, YWHAB, ITGB1BP1, LGALS8 and GNB2L1. This facilitates a particularly specific blocking of the biological activity of the at least one protein cluster (CMP) or group of CMPs. Moreover, the pharmaceutical preparation is also low in side-effects.

In another aspect of the disclosure the pharmaceutical preparation comprises a pharmaceutically acceptable carrier. In the present disclosure the pharmaceutically acceptable carrier depends on the concrete design of the pharmaceutical composition. A person skilled in the art can determine directly which pharmaceutically acceptable carrier can be used in the present disclosure. In one preferred embodiment, the pharmaceutically acceptable carrier is selected from physiological saline. The amount of the pharmaceutically acceptable carrier is generally from 0.1% by weight to 99.9% by weight, preferably from 5% by weight to 95% by weight, more preferably from 40% by weight to 90% by weight, and most preferably from 60% by weight to 80% by weight.

In another aspect of the disclosure the pharmaceutical preparation is formulated for oral, parenteral, intravenous, intramuscular, buccal, transdermal or transmucosal route of administration, in immediate release form or in controlled release form. In this way the pharmaceutical preparation can be administered in a particularly flexible way and can be optimally adapted to the disease to be treated.

The invention in a further aspect relates to a method for identifying a cancerous disease in a patient comprising at least the step of examining if at least a protein cluster (CMP) or a group of CMPs containing at least CD26 and CD29 can be identified on the cell surfaces of cells from a part of the body of the patient, wherein said part is prostate tissue and suspected to be affected by said disease. Further, at least one protein cluster (CMP) or group of CMPs also containing CD44 and/or CD54 and/or CD138 and lacking at least one of CD3, CD4, CD8, CD10, CD13, CD19, CD20, CD38, CD49d, CD58, and CD80 is identified on the cell surfaces of said cells. This represents a reliable indication of the presence of prostate cancer.

The invention in this regard is based upon the insight that CD26 and CD29 within the context of prostate cancer are so-called "lead proteins". On a high level of molecular cell organization, protein clusters are frequently interlocked as protein cluster networks (corresponding to said CMP motifs), which are controlled by said lead proteins: when the lead protein is inhibited, the clusters disassemble leading to loss of function. Therefore, the inventive method allows for easy and reliably identification of a prostate cancer

In its most simple embodiment the method according to the invention it may be tested for the presence of a CMP consisting of CD26 and CD29 only. In principle, though, it may also be tested for CMPs comprising besides CD26 and CD29 also further CDs, proteins and/or molecules, for example one or more CDs and/or molecules as listed in the Supplementary Table (see Annex).

In another aspect of the invention, at least one protein cluster (CMP) or group of CMPs lacking at least one of CD44, CD54, and/or CD138 is identified on the cell surfaces of said cells. This represents a particularly reliable indication of the presence of prostate cancer. The protein cluster or protein group (CMP) in principle may also lack several or all CDs form the group of CD3, CD4, CD8, CD10, CD13, CD19, CD20, CD38, CD49d, CD58, CD80, CD44, CD54, and/or CD138. A reliable indication of the presence of prostate cancer is given in further development of the invention in that the CMP is lacking at least CD44 and/or CD54 and/or CD138.

In another aspect of the invention, the disease is identified as prostate cancer if the cells derive from stroma and/or neoplastic epithelium of acini of prostate tissue and/or if at least 70%, preferably at least 75%, of the CMPs on the cell surface of at least one cell contain CD26 and/or CD29 and/or if at least 20%, preferably at least 35%, of the CMPs on the cell surface of at least one cell comprise CD26 and/or CD29 and lack at least CD3, CD4, CD8, CD10, CD13, CD19, CD20, CD38, CD49d, CD58, and CD80. In this way prostate cancer can be identified in a particularly simple and reliable way in the patient concerned.

Another embodiment of the disclosure provides a method of identifying at least one candidate for development of anti-cancerous or anti-inflammatory agents, comprising at least the step of examining if at least one protein cluster (CMP) or a group of CMPs containing at least CD26 and/or CD29 can be identified on the cell surfaces of cells from a part of the body of the patient, wherein said part of the body is suspected to be affected by a cancerous disease, wherein said part of the body is prostate tissue and the disease is prostate cancer if at least one protein cluster (CMP) or group of CMPs also containing CD44 and/or CD54 and/or CD138 and lacking at least one of CD3, CD4, CD8, CD10, CD13, CD19, CD20, CD38, CD49d, CD58, and CD80 is identified on the cell surfaces of said cells. This allows for a particularly reliable identification of a patient suffering from prostate cancer. The disclosure in this regard is based on the insight that CD26 and/or CD29 within the context of cancerous diseases are so-called "lead proteins". On a high level of molecular cell organization, protein clusters are frequently interlocked as protein cluster networks (corresponding to said CMP motifs), which are controlled by said lead proteins: when the lead protein is inhibited, the clusters disassemble leading to loss of function. Therefore, the inventive method allows for easy and reliably identification of said candidate for development of agents directed against the cancerous disease said part of the body is affected by. In the most simple embodiment of the method according to the invention, it is tested for the presence of a CMP consisting of CD26 and/or CD29 only. In principle, though, it may also be tested for CMPs comprising besides CD26 and/or CD29 further CDs, proteins and/or molecules, for example one or more CDs and/or molecules as listed in the Supplementary Table (see Annex).

Another embodiment of the disclosure provides a composition or kit for use in a method for identifying prostate cancer in a patient, the composition or kit comprising at least one antibody and/or at least one ligand wherein the antibody and/or ligand binds at least one cell surface protein selected from the group of CD3, CD4, CD8, CD10, CD13, CD19, CD20, CD26, CD29, CD38, CD44, CD49d, CD54, CD58, CD80 and CD138 and wherein the antibody and/or ligand is coupled with a label. The composition or kit can be used in one of the aforementioned methods. The composition or kit can also comprise propidium iodide or the like for localizing cell nuclei.

In another aspect of the invention, at least one label is a fluorescent dye, quantum dot, radioactive tag, spin label, binding tag for a second antibody and/or an enzyme. This allows for a flexible adaptation of the composition or kit to various applications and detection methods.

Another embodiment of the disclosure provides a biochip for use in a method according to any of the preceding embodiments wherein at least one surface of the biochip comprises antibodies and/or ligands binding to a protein cluster (CMP) or a group of CMPs of cell surfaces of human and/or animal cells, wherein the CMP contains at least CD26 and/or CD29 and wherein at least one surface of the biochip contains antibodies and/or ligands binding to a protein cluster (CMP) or a group of CMPs of cell surfaces of human and/or animal cells, wherein the CMP contains at least one of CD3, CD4, CD8, CD10, CD13, CD19, CD20, CD38, CD44, CD49d, CD54, CD58, CD80 and CD138. In this way for the identification of cancerous diseases important cell markers can be tested by means of the biochip according to the invention, so that a particularly fast and reliable diagnosis is facilitated. In this regard the disclosure is based on the insight that CD26 and/or CD29 in the context of cancerous diseases are "lead proteins". On a high level of molecular cell organization, protein clusters are frequently interlocked as protein cluster networks (corresponding to said CMP motifs), which are controlled by said lead proteins: when the lead protein is inhibited, the clusters disassemble leading to loss of function. Therefore, the disclosed biochip allows for easy and reliably identification of a cancerous disease. In the most simple embodiment/design of the biochip according to the invention at least one surface may comprise antibodies and/or ligands, by means of which it can be tested for the presence of a CMP consisting of CD26 and/or CD29 only. In principle at least one surface of the biochip may be equipped with antibodies and/or ligands in such a way that it can also be tested for CMPs comprising besides CD26 and/or CD29 further CDs, proteins and/or molecules, for example one or more CDs and/or molecules as listed in the Supplementary Table (see Annex).

Further features of the invention derive from the claims, the embodiments, as well as the drawings and tables. The features and feature combinations previously mentioned in the description as well as the features and feature combinations mentioned in the following in the embodiments can be used not only in the combination indicated in each case, but also in all other combinations or individually, without leaving the scope of the invention. It is shown in:
- Figure 1: a schematic illustration of the topological hierarchies of proteins within a toponome;
- Figure 2: mappings of molecular cell components (MCC) by MELC/TIS running 9 incubation-imaging-bleaching cycles on a single fixed tissue section.
- Figure 3: a toponome map showing the location of the 30 most frequent out of more than 2,000 different CMPs detected in the visual field of Figure 2;
- Figure 4: mappings of molecular cell components by MELC/TIS inside and around a prostate acinus and in the surrounding stroma;
- Figure 5: an illustration of the 41 most frequent out of more than 2,100 different CMPs in the area shown in the MELC/TIS cycle map of Figure 4;
- Figure 6: an illustration of the selective expression of CMP1 in subcellular sites of neoplastic prostate acini; and
- Figure 7: a scheme illustration of the major features of the networks formed by 17 different cell surface proteins, and functional annotation of these networks to specific cell types in prostate cancer.

The microscopic fluorescence robot technology MELC/TIS is capable of imaging at least 100 different molecular cell components (MCCs) in one cell or tissue section. Multi-Epitope-Ligand-Cartography (MELC) /toponome imaging system (TIS) overcomes the spectral limitation of traditional fluorescence microscopy by using large dye-conjugated tag libraries and automatically bleaching a dye after imaging and re-labelling the same or another MCC in the identical sample with the same dye coupled to a tag having the same or another specificity, and repeat similar cycles with other tags for multiple times revealing multidimensional colocation patterns. By this approach, the so called toponome (the functional protein networks, or, the biological code of the cell) of a cell can be gathered. After the first description of the technology in 1990, the relevance of this approach to cell function has been increasingly recognized.

The MELC/TIS technology is unparalleled when it comes to the colocalization of a very large number of proteins using microscopy. The ability to colocalize proteins on a large scale is regarded as a bottleneck in the drive to understand how proteomes are organized in individual cells, and how interlocked clusters of proteins exert control over cellular functions. In the following examples MELC/TIS was used for the analysis of prostate tissue. By cyclical imaging of 17 different MCCs, 16 of which were cell surface proteins, the resulting high-dimensional protein colocation and anti-colocation code is described. Also, cell type specific protein clusters are visualized, and a protein cluster motif that is selectively expressed by neoplastic epithelial cells inside prostate acini is demonstrated.

### Material and Methods

**Specimen.** A single tissue was cut from a prostate tissue block of radical prostatectomy (61 yr old man with lymph node metastasis and histologically confirmed prostate cancer). The tissue block was removed from the peripheral zone of the prostate, containing multicentric foci of cancerous prostate acini and acini with features of low grade and high grade intraepithelial neoplasia (PIN).

**Tag library.** The tags used to label proteins and ligands in the tissue section, assembled as a toponome mapping library (MELC/TIS tag library), are summarized in Table 1. Table 1 shows a list of molecular cell components labelled in the following examples (column 1) together with their locus link annotations (columns 2 and 3) and the corresponding incubation imaging bleaching cycles (column 4). Column 3 gives some of the known cellular expressions and functions of the molecules illustrating why they were selected for the TIS procedure searching for their topological assemblies. The tags used for cyclical imaging procedures were either conjugated to FITC or PE, and signals of two tags per cycle were imaged. The molecules selected for the toponome imaging procedures in this study are all reference markers of the cell surface, which have all been calibrated for MELC/TIS in an earlier study. Some of the known cellular expressions and functions of these molecules are listed in column 3 of table 1 illustrating why these molecules were chosen for the TIS procedure.

**Preparation of the tissue section.** A five µm thick tissue section was cut from the prostate tissue block in a cryotome at -15°C and placed on a large cover slip as described. The section was then fixed in acetone at room temperature (RT) for 10 seconds, air dried and stored at -80°C until use. Before use (see MELC/TIS below), the section was again fixed in acetone (at -20°C) for 10 min, air dried, and then rehydrated in PBS at RT. This was followed by incubation with normal goat serum for 30 min, and a washing step with PBS.

**Optical parameters.** To obtain an overview at 2D on the many cell types and their phenotypic variations present in the tissue, an optical set up was chosen, that was already successfully applied for the detection of protein clusters in other biological samples. For the MELC/TIS experiments (see below) a 16x oil objective with a numerical aperture of 0.5 was applied giving a pixel dimension of 850 nm x 850 nm (= 0.7225 µm) by using a two fold binning.

**MELC/TIS.** The features and functionalities of a MELC/TIS imaging robot have been described earlier. The procedure to run repetitive incubation imaging bleaching cycles (RIIBC) on the tissue section was as follows.

In brief, the cover slip with the tissue section (above) was placed on the stage of an inverted wide-field fluorescence microscope (Zeiss) equipped with fluorescence filters for fluoresceine-iso-thio-cyanate (FITC) and phycoerythrin (PE) (filter sets for FITC: excitation 480/40 BP; beam splitter 505 LP; emission 527/30 BP; filter sets for PE: excitation 546/11 BP; beam splitter 560 LP; emission 585/40 BP). For the cyclical imaging procedures, a given FITC- and PE-conjugated tag was merged in one cycle (Table 1, column on the right). Altogether 9 cycles were performed. Fluorochrome-labelled tags and wash solutions were added and removed robotically under temperature control (20°C), avoiding any displacement of the sample and objective. In each cycle, two different tags, conjugated to either FITC or PE, were added (cycle sequence see table 1); phase contrast and fluorescence images were acquired by a high-sensitivity cooled CCD camera; the sample was washed with PBS and bleached at the excitation wavelengths; and post-bleaching phase contrast and fluorescence images were acquired. Data acquisition was fully automated using home made software. Note that interference of antibodies was avoided by permuting the cycle position of antibodies for optimal labelling results during the calibration procedure preceding the definitive measurements. The definitive sequence of cycles and molecules/proteins labelled per cycle is given in table 1. The sensitivity of the approach ranges between 500 and several thousand copies of the molecules labelled per cell, depending on the avidity of the used tags. Since monoclonal CD antibodies used in this study are extremely well characterized for their specificity in diagnostic medicine, we expect that positive signals indeed represent the molecules of interest. Hence false positive results can be excluded. We can not exclude that tissue sites, in which no signal is seen, do not contain the molecule of interest, which can be due to epitope masking resulting from conformational changes or binding of the corresponding epitope to interacting proteins.

**Construction of a two-dimensional toponome map.** Fluorescence images, produced by each tag, were aligned pixel-wise using the phase contrast images, with an in- register accuracy of +/- one pixel. Fluorescent pixels were then parsed by regarding the list of fluorescence intensities I₁, I₂, I₃...Iₙ for proteins 1, 2, 3...n in any particular pixel or voxel as the values of an n-dimensional vector associated with that pixel. This vector can then be binarized by selecting thresholds T₁, T₂, T₃...Tₙ for proteins 1, 2, 3...n, and setting the vector values for any protein m to zero if Iₘ < Tₘ and to 1 if not, using thresholds manually set by human experts from within an automatically generated range. The binarized images were then combined to form a list of combinatorial molecular phenotypes (CMPs) representing the proteins/molecules expressed in each pixel, or groups of CMPs representing regions of interest. Given CMPs (protein clusters) or groups of CMPs were visualized in false colours at their location. We have established these steps in home made software, termed MoPPi (Modular Processing Pipeline).

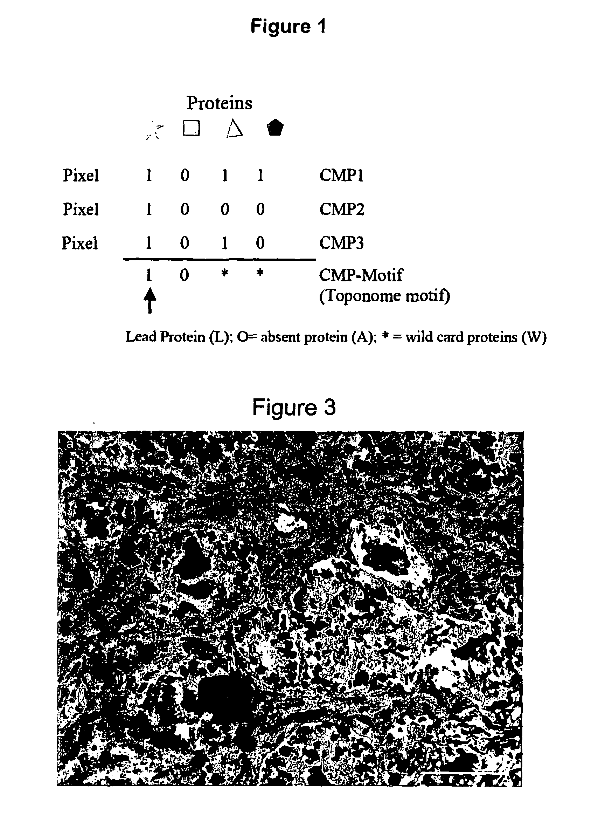

Each signal within a CMP is mapped as tag present (=1) or tag absent (=0), depending on whether the value for the fluorescence signal is above or below this threshold, respectively (=1 bit information per protein at a pixel/voxel). CMPs assembled as a group will have unique features as defined by the assembly's lead proteins (L=1), absent proteins (A = anticolocated, 0) and wild card proteins (W = variably occurrence of a protein 0 and 1 in given CMPs of a CMP group). We define such CMP groups as a CMP motif (Figure 1), denoting a given functional region of a cell or tissue. Figure 1 shows a schematic illustration of the topological hierarchies of proteins within the toponome. By using a three symbol code (LAW, Figure 1), differences of cell states and cell types can be readily identified in studies comparing large experiments, or, diseases with normal conditions. "L" means lead protein (common to all CMPs of a CMP motif), "A" means absent protein (absent in all CMPs of a CMP motif), and "W" means wild card proteins (proteins that are variably associated with the (L) and the (A) proteins of a motif) (source: Ref 7).

In the present set up, with the optical parameters chosen (see material and methods section), a pixel expressing a given CMP, or protein cluster, has a dimension of 0.7225 µm.

### Results

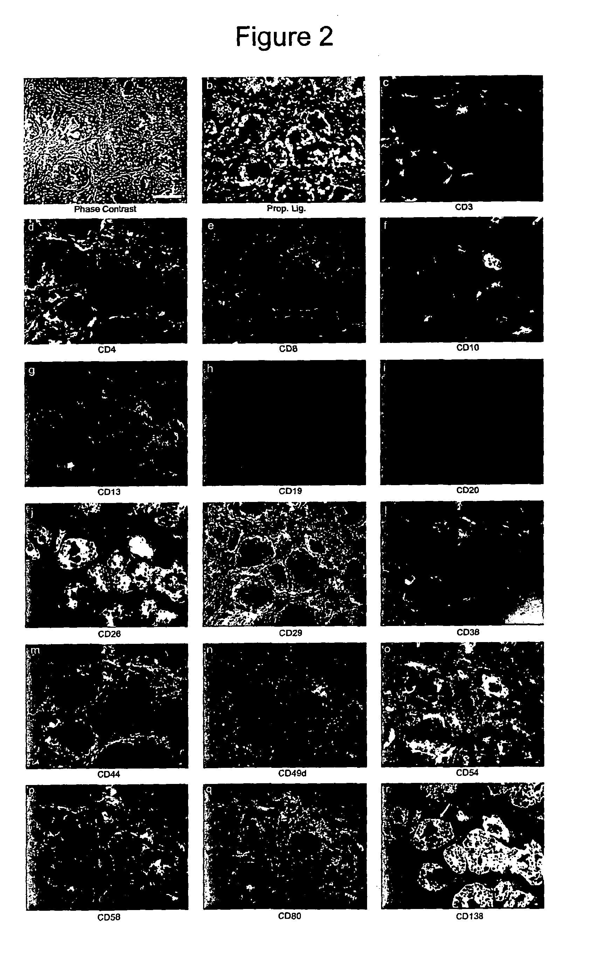

**Nine MELC/TIS cycles generate a gallery of 17 images.** To illustrate working of toponomics in prostate cancer analysis, we have chosen a single tissue section of histologically verified prostatic cancer presenting as acinus type, with multifocal variations including presence of intraepithelial neoplasia (PIN). The results described henceforth were obtained by using the robot system MELC/TIS for the imaging of the toponome. To select a visual field for our study, a 16x oil phase contrast/fluorescence objective was used. Once the visual field was selected by an expert (Figure 2 a), we started the fully automated procedure of cyclical imaging of 17 different affinity tags in the selected visual field (Figure 2 b-r). This tag library had been calibrated by a procedure described by us previously. Interference of antibodies was avoided by permuting the cycle position of antibodies for optimal labelling results during the calibration procedure preceding the definitive measurements. The definitive sequence of cycles and molecules/proteins labelled per cycle is given in table 1. The sensitivity of the approach ranges between 500 and several thousand copies of the molecules labelled per cell, depending on the avidity of the used tags. Since the tags used in this study, especially the monoclonal CD antibodies are extremely well characterized in the literature for their specificity of the molecules labelled *in situ*, we expect that positive signals indeed represent the molecules of interest. Hence false positive results are very unlikely, while we can not exclude that tissue sites, in which no signal is seen, do not contain the molecule of interest, which can be due to epitope masking resulting from conformational changes or binding of the corresponding epitope to interacting proteins.

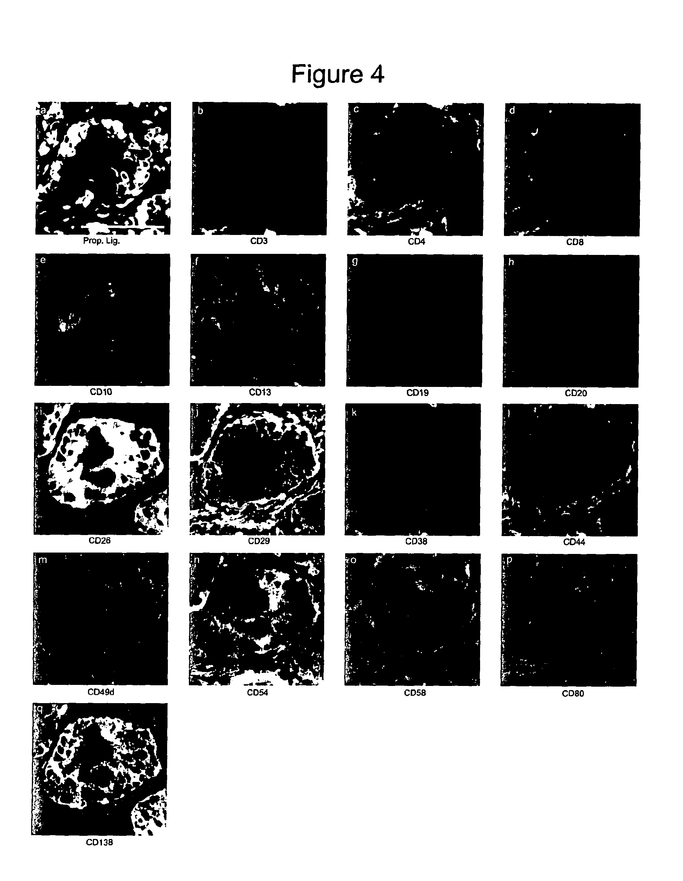

Figure 2 generally shows mappings of molecular cell components (MCC) by MELC/TIS (tag library of table 1) running 9 incubation-imaging-bleaching cycles on a single fixed tissue section. Figure 2 a shows phase contrast view followed by MELC/TIS maps of 17 proteins/ molecules (shown in Figure 2 b-r). For specificity and selectivity of the labelled MCC see table 1. The prostate acinus denoted by an arrow in (r), is magnified in a corresponding gallery of images of Figure 4 (Bar: 100 µm). As illustrated in Figure 2, the imaging robot generated 17 single grey value images resulting from 9 different incubation-imaging-bleaching cycles. To correlate our results with known markers for prostate cancer classification, we have included antibodies against CD26 specifically recognizing prostate epithelium and CD138 as a marker for prostate cancer progression. As illustrated in Figure 2 (j, r), these antibodies selectively label prostate epithelium, which is in good agreement with earlier findings. Some of the tags (CD3, CD4, CD8, CD38) selectively recognized cell structures in the fibrocollagenous stroma surrounding the prostate acini (Figure. 2). Presence of CD4 and CD8 T lymphocytes (Figures 2d, e, respectively) substantiates presence of abundant inflammatory cells. Other tags labelled both cell structures in the stroma and inside the epithelial acini, such as CD13, CD29, CD44, CD49d, CD54, CD58, and CD80 (Figures 2, g, k, m, n, o, p, q, respectively). To localize cell nuclei, we have included propidium iodide in our imaging cycles (Figure. 2 b). CD20 and CD19 as markers of B lymphocytes were negative (Figure 2 h, i, respectively). Interestingly, CD10 was present in a subset of epithelial cells inside some of the acini (Figure 2 f).

**Localization of protein clusters (CMPs).** As described in the material and methods section, we have applied our MoPPi software tool to (i) align the images from Figure 2, (ii) set expert-selected thresholds for signal binarization as described, and (iii) use our CMP extraction algorithm to find all CMPs contained in the corresponding visual field. This procedure led to what we term a toponome map. Altogether we detected 2,100 different mutually exclusive CMPs. Supplementary Table 1 gives 165 CMPs in the order of their frequency (number of pixels expressing these CMPs) and shows the most frequent CMPs detected in the visual field of Figure 2. The CMPs are based on the combinatorial analysis per pixel of 16 different cell surface protein signals (table 1), that do not colocalize with propidium iodide signal. CMPs are sorted according to their pixel frequency (from top to bottom, high to low).

Table 2 shows a coding list of the 30 most frequent distinct CMPs (out of 2,100 in total) detected in the visual field of Figure 2. The frequency of a CMP is defined as the number of pixels in the visual field of Figure 2, which express this given CMP. Hence, each CMP is distinct from other CMPs (compare the combinatorial 0/1 codes, horizontal lines in table 2); moreover, the pixels that express a given CMP, exclude expression of any other distinct CMP, so that CMPs are, topologically, mutually exclusive.

The expression/location of these CMPs in the tissue is shown in Figure 3 as overlay with the CD138 fluorescence signal. In other words, Figure 3 illustrates the location of these CMPs by aligning their different colours (resp. grey scales) with the original CD138 fluorescence signal. The colours/grey scales for the distinct CMPs on the left correspond to the colours indicating their location in Figure 3. The column on the right (frequency) indicates the number of pixels that express the given CMP. Protein/molecule coding list (numbers in the top line): 1 = propidium iodide; 2 = CD3; 3 = CD4; 4 = CD8; 5 = CD10; 6 = CD13; 7 = CD19; 8 = CD20; 9 = CD26; 10 = CD29; 11 = CD38; 12 = CD44; 13 = CD49d; 14 = CD54; 15 = CD58; 16 = CD80; 17 = CD138. The range of CMP frequencies between 24.087 and 873 pixels (column on the right), with a cut off value at 873 pixels (corresponding to 30 different CMPs, column on the left) was chosen, because frequencies below 800 are difficult to detect visually in an image. This allowed for the examination by visualization, (i) which CMPs are restricted to the CD138 positive epithelium of acini, (ii) which CMPs are confined to the stroma, and (iii) which CMPs are expressed by both stroma and the epithelium.

**Cell type specific protein clusters and protein cluster motifs.** To provide a more detailed view of these CMP locations, a detail of Figure 2 (r, arrow) was depicted: acinus singled out by CD138. This detail is displayed as a selected gallery of the 17 images corresponding to the 9 TIS cycles (Figure 4). Figure 4 shows mappings of molecular cell components by MELC/TIS inside and around a prostate acinus and in the surrounding stroma. The area corresponds to the detail of Figure 2 (r, arrow). Figure 4 a shows a fluorescence signal of propidium iodide localizing cell nuclei; Figures 4 b-q show localizations of 16 different cell surface proteins specified in table 1. (Bar: 100 µm)

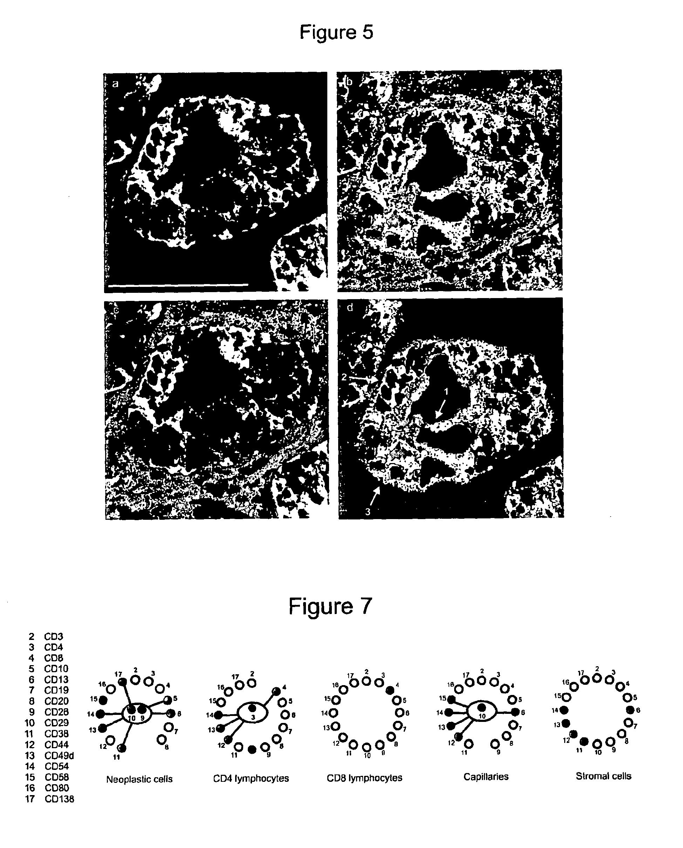

The most frequent CMPs of this area were then localized in different colours and as overlays with the CD138 fluorescence signal (Figures 5 b, c, d). Figure 5 generally illustrates the 41 most frequent out of more than 2,100 different CMPs in the area shown in the MELC/TIS cycle map of Figure 4: Figure 5 a shows a fluorescence signal of CD138; Figure 5 b shows 41 most frequent CMPs displayed in different colours; Figure 5 c shows a fraction of the latter CMPs selectively expressed by cellular structures in the stroma surrounding the acinus; and Figure 5 d shows CMPs which are selectively expressed by a fraction of epithelial cells inside the acinus showing features of proliferative intraepithelial neoplasia (PIN). Figure 5 d also shows irregular spacing and stratification of the inner secretory cells with cytoplasmic projections towards the lumen of the acinus (d, arrow 1); basal cell layer with partially inconspicuous (d, arrow 2), and partially discontinuous parts (d, arrow 3). The basal epithelial cells (d, arrow 2) displaying normal cell features do not express any of these CMPs.

The colour coding (resp. grey scale) list for CMPs of Figure 5 c and 5 d is given in Table 3 part I and part II, respectively: part I denotes CMPs which are selective for the stroma (Figure 5 c), and part II gives the CMPs selective for PIN cells (Figure 5 d). The latter CMPs represent a group that together are assembled as a CMP motif with CD26 and CD29 as lead proteins (compare table 3, part II; Bar: 100 µm.)

Table 3 (part I and part II) gives the corresponding colour or grey scale coding list of these CMPs. Table 3 part I shows a colour (resp. grey scale) coding list of the 25 most frequent CMPs (out of 2,100) which are selectively expressed by cell structures in the stroma surrounding neoplastic prostate acini. The CMPs are sorted according to their frequency (from top to bottom, high to lower) and expression of these CMPs in the stroma is shown in Figure 5 c as overlay with the CD138 signal. Table 3 part II shows a colour (resp. grey scale) coding list of the 16 most frequent CMPs (out of 2,100) which are selectively expressed by cell structures in the neoplastic prostate acinus. The CMPs are sorted according to their pixel frequency (from top to bottom, high to lower). Expression of these CMPs in the acinus is shown in Figure 5 d as overlay with the CD138 signal.

In other words, Figure 5 c and Figure 5 d show those CMPs which are restricted either to the stroma or the CD138 positive acinus, respectively. This acinus shows features of proliferative intraepithelial neoplasia (PIN), as indicated (among other things) by (i) irregular spacing and stratification of the inner secretory cells with cytoplasmic projections towards the lumen of the acinus (Figure 5 d, arrow 1), and (ii) the basal cell layer with partially inconspicuous (Figure 5 d, arrow 2), and partially discontinuous parts (Figure 5 d, arrow 3).

Notably, 25 CMPs are selectively expressed by cells in the stroma (Table 3, part I; Figure 5 c), and 16 CMPs (Table 3 part II; and Figure 5 d) are unique for a fraction of epithelial cells displaying the features of PIN. Interestingly, many of the basal epithelial cells, displaying CD138, do not express these CMPs (Figure 5 d, arrow 2). This strongly suggests that neoplastic cells and inconspicuous epithelial cells can be distinguished by the CMPs shown in Table 3 (part II). Moreover, these CMPs together represent a CMP group that fulfil the criteria of a CMP motif (compare Table 3 part II with the scheme in Figure 1): All CMPs shown in table 3 part II, have in common CD26 and CD29 as lead proteins (= L), while CD3/CD4/CD8/CD19/CD20/CD44/CD80, are absent (anti-colocated) in all these CMPs, and CD10/CD13/CD38/CD49d/CD54/CD58/CD138 are variably associated with the lead and anti-colocated molecules (= 0). As shown and described in detail for the human immune system and other cell systems, this gives a three symbol code (L, A, W) for protein clusters selectively associated with neoplastic cells in the present example (Table. 3 part II: CMP motif).

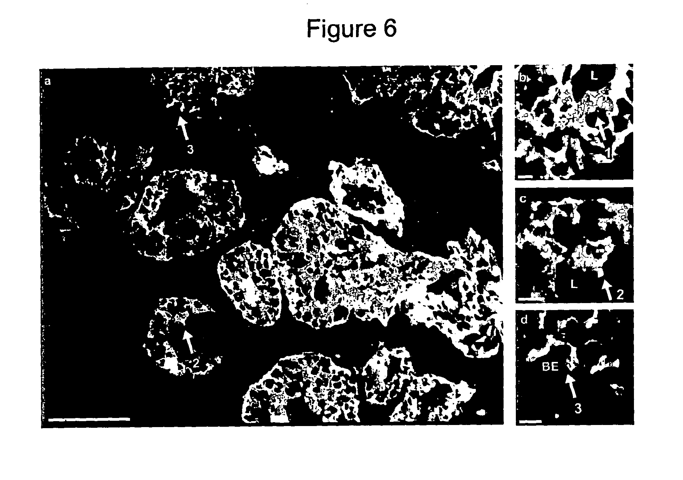

By following a strategy of extraction of the most prominent and relevant information contained in the many CMPs detected, each single CMP out of the 30 and 41 most frequent CMPs has been visualized. The CMPs are summarized in Tables 2 and 3, and in supplementary table 1, respectively. As shown in table 3 (part II), the most frequent CMPs within the PIN-CMP motif is CMP1 coexpressing the lead cell surface proteins CD29/CD26 together with CD54 and CD138, while all other 12 cell surface proteins are anti-colocated (= 0) in that CMP. This CMP is indeed expressed in all except one of the acini undergoing neoplasia in the visual field (Figure 6).

Figure 6 illustrates the selective expression of CMP1 in subcellular sites of neoplastic prostate acini. CMP1 is the most frequent CMP within the CMP motif of table 3, part II. Figure 6 a shows an overview with arrow 1 and arrow 2 denoting apical sites of secretory cells, and arrow 3 denoting cell projections at the basolateral site of an acinus. Figures 6 b-c show details illustrating tissue sites denoted by arrows 1, 2 and 3, respectively. Note the circumscribed association of the CMP1 (brown colour) with cell surface fluorescence signal of CD133 (white). "BE" means basal epithelial cell layer. (Bars: 6 a: 100 µm; 6 b-d: 10 µm.)

As indicated by arrows, the location of this CMP is interesting, because it singles out apical (Figure 6a, arrow 1, arrow 2) and/or basolateral sites of the secretory cells, or, epithelial cells which are close to the basal membrane, or which have formed cell projections that might have penetrated the basal lamina border (Figure 6 a, arrow 3). Hence, while this CMP is clearly present in most of the acini in the field, it is expressed selectively at subcellular sites of the epithelial cells which are suspicious for cancer progression (migration/invasion).

Another prominent feature of the tissue analyzed in the present example is the striking selectivity of CMPs for non-epithelial cellular structures in the fibrocollagenous stroma surrounding the acini (Figure 5 c). Table 3 (part I) gives the colour coding list for these CMPs. These structures could be classified as T lymphocytes (CD4 and CD8) (table 3 part I, T4, and T8, respectively), capillary endothelium (CD29) (table 3 part I, C), and other stromal cells which were not further characterized (S) (table 3 part I).

Together these major results are summarized in a scheme (Figure 7) illustrating the cell type specific protein clusters in prostate tissue. Figure 7 shows a scheme illustrating the major features of the networks formed by 17 different cell surface proteins (coding list, on the left), and functional annotation of these networks to specific cell types in prostate cancer. Networks are extracted from the toponome motifs shown in tables 1 through 3. Proteins co-mapped by MELC/TIS are arranged as circles around cell type specific lead proteins; Solid dots represent proteins, which are variably associated with the lead proteins (corresponding to the wild card proteins illustrated in Figure 1); Empty dots represent proteins which are anti-colocated (= 0 in Figure 1). Lead proteins are encircled (very center of each network), and their differential assembly with the wild card protein is symbolized by lines. Toponome motifs were only found for PIN-, CD4 lymphocytes and capillary cells, while stromal cells and CD8 lymphocytes did not show any motif within the most frequent CMPs of tables 1 through 3. Propidium iodide as DNA marker (number 1 in the molecule coding list) is omitted because of its irrelevant information concerning cell surface protein clustering.

To sum up, these findings describe the feasibility to map a fraction of the cell surface toponome of proteins in a single tissue section in this disease. Accordingly, the present findings provide insight in protein networks driving the disease. The present approach has led to entirely structure-bound combinatorial data generating a new type of high-dimensional data space. Mapping the organized proteome by MELC/TIS in earlier studies revealed the structure-bound architecture of functional protein networks (the toponome). It has unravelled new cellular mechanisms and lead proteins controlling pathogenic networks in rhabdomyosarcoma cells. By colocalizing 17 different molecular components in one tissue sectionmore than 2,000 different protein clusters (CMPs) were detected, some of which are specifically associated as CMP motifs with the features of neoplasia (e.g. proliferative intraepithelial neoplasia, PIN). Also, CMP motifs selectively associated with inflammatory CD4 lymphocytes and capillary endothelium were detected. No specific motifs were found for stromal cells of the fibrocollagenous stroma surrounding prostate acini, and for inflammatory CD8 lymphocytes, while both these latter cell types did express several single marker proteins. Simple protein network graphs illustrate how these distinct features of differentially associated proteins are selectively displayed by these cell types (Figure 7).

These findings are in keep with our earlier observations, that CMP mapping rapidly leads to insight into relevant protein assemblies and the rules of their topological order, to address what we term the generative grammar of the toponome. Detection of clear-cut cell-type specific clustering of proteins *in situ* relies on the ability of MELC/TIS to co-map a quasi random number of proteins by cyclical imaging in any given data point of a digitized image of a tissue section in one procedure. Compared to the localization of only one or few proteins, this leads to an exponential increase of biological information, as shown in the present invention. Protein clusters (differential local combination of n proteins), rather than a single protein species, clearly distinguish cell types, subcellular sites, or, different functional states of a cell or a tissue. Moreover, on an even higher level of molecular cell organization, protein clusters are frequently interlocked as protein cluster networks (corresponding to CMP motifs), which are controlled by lead proteins: when the lead protein is inhibited, the clusters disassemble leading to loss of function. In the present invention a CMP motif has been found that is expressed inside prostate acini by epithelial cells with features of neoplasia (e.g. proliferative intraepithelial neoplasia, PIN) (PIN specific motif), while basal epithelial cells with inconspicuous features do not express this motif. This finding supports the toponome approach to new molecular pathways in prostate cancer.

First, the two lead proteins (CD29 and CD26) of the PIN-specific motif detected here are structurally and functionally distinct: CD29 is a beta integrin chain which associates as a heterodimer non-covalently with the alpha integrin chain subunit. The molecule is involved in cell-cell and cell-matrix interactions. The highly conserved cytoplasmic domain of CD29 interacts with the cytoskeleton. CD26 (dipeptidyl peptidase IV, DPPIV), is a type II transmembrane integral protein. It is a cell surface protease cleaving dipeptides from NH2-termini of proteins provided that the penultimate residue is proline. The distinct structure and function of these two proteins coexisting as lead proteins in all CMPs, or, protein clusters of the detected PIN motif, is consistent with our earlier observation in rhabdomyosarcoma cells, that supramolecular cell surface clusters are, by a rule, composed of highly dissimilar proteins.

Second, the detection of CD29 and CD26 and their corresponding motif singles out these proteins as candidates for therapeutic intervention in prostate cancer. Therefore, a blockade of these proteins, for example by cross-linking agents, interferes with the control of these proteins over the PIN motif, leading to its disassembly.

Third, the CMP motif clusters appear to be specific modules of the cell surface of PIN cells: by interaction with the cytoskeleton, for example through CD29, and through continuous proteolytic activity of CD26, the cluster is involved in PIN-specific cell polarization and migration. An intervention on the level of these two proteins therefore interferes with tumor progression. Given CD26 as a proteolytic lead protein enzyme, assembled with CD29 as an integrin in our present example, a new functional model emerges: cell surface enzymes are important lead elements interacting with cytoskeleton-linking proteins to exert control over topology and function of extended protein cluster networks.

Together, finding of tumour specific targets or target clusters, and understanding the molecular mechanisms of disease *in situ* is a major goal in cancer research. High-throughput transcriptomics and proteomics studies, based on *ex vivo* analyses, have already assembled many new information on interesting target candidates, and efficient methods for handling such high-dimensional data have been described. By contrast, the present invention addresses the architecture of protein networks directly *in situ* in the individual cell or tissue section. This leads to a different kind of high-dimensional data, with a topological mosaic of protein colocation and anti-colocation clusters. Construction of corresponding toponome reference maps of prostate cancer serves as the starting point in the search of disease-specific protein networks and lead proteins. Such reference maps can be used as a scaffold, or, assembly of combinatorial molecular landmarks, for the construction of much larger maps involving many more proteins.

The optical set up in the present study, with a pixel dimension of 0.7225 µm, was chosen to address in one visual filed as many cells and acini as possible, with a sufficient resolution to find protein clusters associated with cells and cell surfaces. However, if needed, the optical resolution of MELC/TIS can be substantially enhanced for any kind of analysis pinpointing protein clusters in pixels with a dimension of at least 0.0466 µm, as shown in detail in our earlier 2D and 3D studies.

The foregoing description of various aspects of the invention has been presented for purposes of illustration and description. It is not intended to be exhaustive or to limit the invention to the precise form disclosed, and modifications and variations are possible. Such modifications and variations are intended to be included within the scope of the invention as defined by the accompanying claims. The parameter values for the definition of processing and measuring conditions for the characterization of specific properties of the subject matter of the invention are to be regarded as comprised within the scope of the invention also within the limits of deviations - e.g. due to measuring errors, system errors, weighing errors, DIN tolerances and the like indicated in the documents.

**TABLE 1**

| **Molecule / moity recognized** | **Official symbol** | **Cellular expression / Known Function** | **MELC / TIS robot cycles** |
|---|---|---|---|
| CD4 | [CD4] | CD4 antigen (p44) is expressed by MHC class II-restricted T cells and binds MHC class II molecules, acting as co-receptor in recognition of foreign antigen [Homo sapiens] | cycle 1 PE |
| CD8 | [CD8] | CD8 antigen (alpha polypeptide, p32) is expressed by MHC class I-restricted T cells and a co-receptor for MHC class I-restricted TCRs in antigen recognition [Homo sapiens] | cycle 1 FITC |
| CD3 | [CD3E] | CD3E antigen (epsilon polypeptide, TiT3 complex) is expressed by T cells and part of the TCR receptor complex. It is necessary for cell surface expression of TCR and for antigen recognition and signal transduction. [Homo sapiens] | cycle 2 PE |
| CD26 | [DPP4] | Dipeptidylpeptidase 4 (CD26, adenosine deaminase complexing protein2) is expressed by activated T cells and cleaves off N-terminal X-Pro or X-Ala dipeptides from polypeptides and is a costimulatory molecule in T cell activation. [Homo sapiens] | cycle 2 FITC |
| CD29 | [ITGB1] | Integrin, beta 1 (fibronectin receptor, beta polypeptide, antigen CD29 includes MDF2, MSK12) is expressed by all leukocytes and forms heterodimers with many integrin α subunits which mediate cell-cell and cell-matrix adhesion. [Homo sapiens] | cycle 3 PE |
| CD49d | [ITGA4] | Integrin, alpha 4 (antigen CD49D, alpha 4 subunit of VLA-4 receptor) is an adhesion molecule of T and B cells and associates with β1 or β7. It is involved in lymphocyte migration, homing and rolling, and is a costimulatory molecule for T cell activation. [Homo sapiens] | cycle 3 FITC |
| CD138 | [SDC1] | Syndecan 1 is expressed by immature B cells and bears both heparin sulphate and chondroitin sulphate. It links the cytoskeleton to the interstitial matrix. [Homo sapiens] | cycle 4 PE |
| CD13 | [ANPEP] | alanyl (membrane) aminopeptidase (aminopeptidase N, aminopeptidase M, microsomal aminopeptidase, CD13, p150) is expressed by granulocytes and monocytes and their precursors. It | cycle 4 FITC |
| | | cleaves single amino acids from amino terminus of small peptides. It participates in trimming peptides bound to MHC class II molecules. [Homo sapiens] | |
| CD38 | [CD38] | CD38 antigen (p45) is expressed by early-stage B and T cells and has multiple enzymatic activities. It plays possible signalling role in cell activation, proliferation, apoptosis, due to modulation of cytoplasmatic calcium fluxes and phosphorylation of key substrates. [Homo sapiens] | cycle 5 PE |
| CD44 | [CD44] | CD44 antigen is expressed by most cell types and is an adhesion molecule, which mediates leukocyte attachment to and rolling on endothelial cells, homing to peripheral lymphoid organs and sites of inflammation. [Homo sapiens] | cycle 5 FITC |
| CD54 | [ICAM1] | Intracellular adhesion molecule 1 (CD54) is expressed by activated T and B cells and binds CD11a/CD18 (LFA-1) and CD11b/CD18 (Mac-1), contributing to leukocyte extravasation from blood vessels. [Homo sapiens] | cycle 6 PE |
| CD58 | [CD58] | CD58 antigen (lymphocyte function-associated antigen 3) is expressed by many hematopoietic and non-hematopoietic cells and mediates the adhesion between killer T cells and target cells, antigen-presenting cells and T cells, thymocytes and thymic epithelial cells. [Homo sapiens] | cycle 6 FITC |
| CD20 | [MS4A1] | Membrane-spanning 4-domains, subfamily A, member 1 is expressed by B lineage cells and plays a possible role in regulation of B cell activation and proliferation. There is evidence for CD20 function as a B cell calcium channel subunit. [Homo sapiens] | cycle 7 PE |
| CD10 | [MME] | Membrane metallo-endopeptidase (neutral endopeptidase, enkephalinase, CALLA, CD10) is expressed by pre-B and pre-T cells and is a marker for common ALLs and certain lymphomas. It cleaves at the amino side of hydrophobic residues and inactivates several bioactive peptides. [Homo sapiens] | cycle 7 FITC |
| CD19 | [CD19] | CD19 antigen is expressed by B lineage cells and is a critical signal transduction | cycle 8 PE |
| | | molecule that regulates B cell development, activation and differentiation. It regulates responses, playing a dominant role in establishing signalling thresholds for antigen receptors and other surface receptors on B lymphocytes. [Homo sapiens] | |
| CD80 | [CD80] | CD80 antigen (CD28 antigen ligand 1, B7-1 antigen) is expressed by activated B and T cells. It is responsible for Co-stimulation of T cell activation with CD86. It plays a potential role in autoimmune, humoral and transplant responses. [Homo sapiens] | cycle 8 FITC |
| Prop lig. | - | Propidium iodide is an intercalating agent and a fluorescent molecule, that can be used to stain DNA nucleic acids. | cycle 9 |

**Table 2**

| **proteins / molecules** | | | | | | | | | | | | | | | | | | | |
|---|---|---|---|---|---|---|---|---|---|---|---|---|---|---|---|---|---|---|---|
| | | **1** | **2** | **3** | **4** | **5** | **6** | **7** | **8** | **9** | **10** | **11** | **12** | **13** | **14** | **15** | **16** | **17** | **freq.** |
| 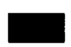 | CMP 1 | 0 | 0 | 0 | 0 | 0 | 0 | 0 | 0 | 0 | **1** | 0 | 0 | 0 | 0 | 0 | 0 | 0 | 24.087 |
| 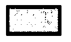 | CMP 2 | 0 | 0 | 0 | 0 | 0 | 0 | 0 | 0 | **1** | **1** | 0 | 0 | 0 | **1** | 0 | 0 | 1 | 6.671 |
| 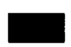 | CMP 3 | 0 | 0 | 0 | 0 | 0 | 0 | 0 | 0 | 0 | **1** | 0 | 0 | 0 | **1** | 0 | 0 | 0 | 5.935 |
| 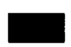 | CMP 4 | 0 | 0 | 0 | 0 | 0 | 0 | 0 | 0 | 0 | 0 | 0 | 1 | 0 | 0 | 0 | 0 | 0 | 5.623 |
| 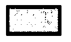 | CMP 5 | 0 | 0 | 0 | 0 | 0 | 0 | 0 | 0 | **1** | **1** | 0 | 0 | 0 | 0 | 0 | 0 | 0 | 5.271 |
| 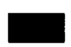 | CMP 6 | 0 | 0 | 0 | 0 | 0 | 0 | 0 | 0 | **1** | 0 | 0 | 0 | 0 | 0 | 0 | 0 | 0 | 4.779 |
| 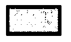 | CMP 7 | 0 | 0 | 0 | 0 | 0 | 0 | 0 | 0 | **1** | **1** | 0 | 0 | 0 | **1** | 0 | 0 | 0 | 4.434 |
| 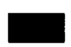 | CMP 8 | 0 | 0 | 0 | 0 | 0 | 0 | 0 | 0 | 0 | **1** | 0 | **1** | 0 | 0 | 0 | 0 | 0 | 4.074 |
| 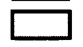 | CMP 9 | 0 | 0 | 0 | 0 | 0 | 0 | 0 | 0 | 0 | 0 | **1** | 0 | 0 | 0 | 0 | 0 | 0 | 3.831 |
| 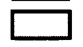 | CMP10 | 0 | 0 | 0 | 0 | 0 | 0 | 0 | 0 | 0 | 0 | 0 | 0 | 0 | **1** | 0 | 0 | 0 | 3.576 |
| 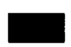 | CMP11 | 0 | 0 | 0 | 0 | 0 | 0 | 0 | 0 | 0 | **1** | 0 | 0 | **1** | 0 | 0 | 0 | 0 | 3.501 |
| 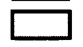 | CMP12 | 0 | 0 | **1** | 0 | 0 | 0 | 0 | 0 | 0 | **1** | 0 | 0 | 0 | 0 | 0 | 0 | 0 | 2.522 |
| 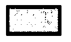 | CMP13 | 0 | 0 | 0 | 0 | 0 | 0 | 0 | 0 | 0 | **1** | 0 | **1** | 0 | **1** | 0 | 0 | 0 | 2.383 |
| 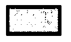 | CMP14 | 0 | 0 | 0 | 0 | 0 | 0 | 0 | 0 | **1** | **1** | 0 | 0 | 0 | 0 | 0 | 0 | **1** | 1.776 |
| 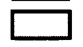 | CMP15 | 0 | 0 | 0 | 0 | 0 | 0 | 0 | 0 | **1** | 0 | 0 | 0 | 0 | **1** | 0 | 0 | 0 | 1.686 |
| 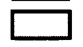 | CMP16 | 0 | 0 | 0 | 0 | 0 | 0 | 0 | 0 | 0 | **1** | 0 | 0 | **1** | **1** | 0 | 0 | 0 | 1.572 |
| 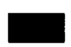 | CMP17 | 0 | 0 | 0 | 0 | 0 | 0 | 0 | 0 | 0 | **1** | **1** | 0 | 0 | 0 | 0 | 0 | 0 | 1.540 |
| 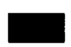 | CMP18 | 0 | 0 | **1** | 0 | 0 | 0 | 0 | 0 | 0 | 0 | 0 | 0 | 0 | 0 | 0 | 0 | 0 | 1.387 |
| 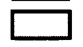 | CMP19 | 0 | 0 | 0 | 0 | 0 | **1** | 0 | 0 | 0 | 0 | 0 | 0 | 0 | 0 | 0 | 0 | 0 | 1.360 |
| 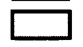 | CMP20 | 0 | 0 | 0 | 0 | 0 | 0 | 0 | 0 | **1** | **1** | 0 | 0 | **1** | **1** | 0 | 0 | **1** | 1.322 |
| 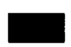 | CMP21 | 0 | 0 | 0 | 0 | 0 | 0 | 0 | 0 | **1** | 0 | 0 | 0 | 0 | **1** | 0 | 0 | **1** | 1.317 |
| 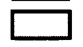 | CMP22 | 0 | 0 | 0 | 0 | 0 | 0 | 0 | 0 | 0 | **1** | 0 | **1** | **1** | **1** | 0 | 0 | 0 | 1.276 |
| 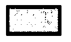 | CMP23 | 0 | 0 | 0 | 0 | 0 | **1** | 0 | 0 | 0 | **1** | 0 | 0 | 0 | 0 | 0 | 0 | 0 | 1.220 |
| 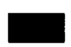 | CMP24 | 0 | 0 | 0 | 0 | 0 | 0 | 0 | 0 | **1** | **1** | **1** | 0 | 0 | **1** | 0 | 0 | **1** | 1.069 |
| 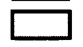 | CMP25 | 0 | 0 | 0 | 0 | 0 | 0 | 0 | 0 | 0 | 0 | **1** | **1** | 0 | 0 | 0 | 0 | 0 | 1.068 |
| 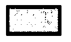 | CMP26 | 0 | 0 | 0 | 0 | **1** | 0 | 0 | 0 | **1** | 0 | 0 | 0 | 0 | 0 | 0 | 0 | 0 | 1.038 |
| 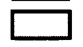 | CMP27 | 0 | 0 | 0 | 0 | 0 | 0 | 0 | 0 | **1** | 0 | 0 | 0 | 0 | 0 | 0 | 0 | **1** | 955 |
| 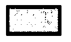 | CMP28 | 0 | 0 | 0 | 0 | 0 | 0 | 0 | 0 | 0 | **1** | 0 | 0 | 0 | **1** | **1** | 0 | 0 | 886 |
| 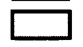 | CMP29 | 0 | 0 | 0 | 0 | 0 | 0 | 0 | 0 | 0 | **1** | 0 | 0 | 0 | 0 | **1** | 0 | 0 | 875 |
| 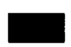 | CMP30 | 0 | 0 | 0 | 0 | 0 | 0 | 0 | 0 | 0 | 0 | 0 | **1** | 0 | **1** | 0 | 0 | 0 | 873 |

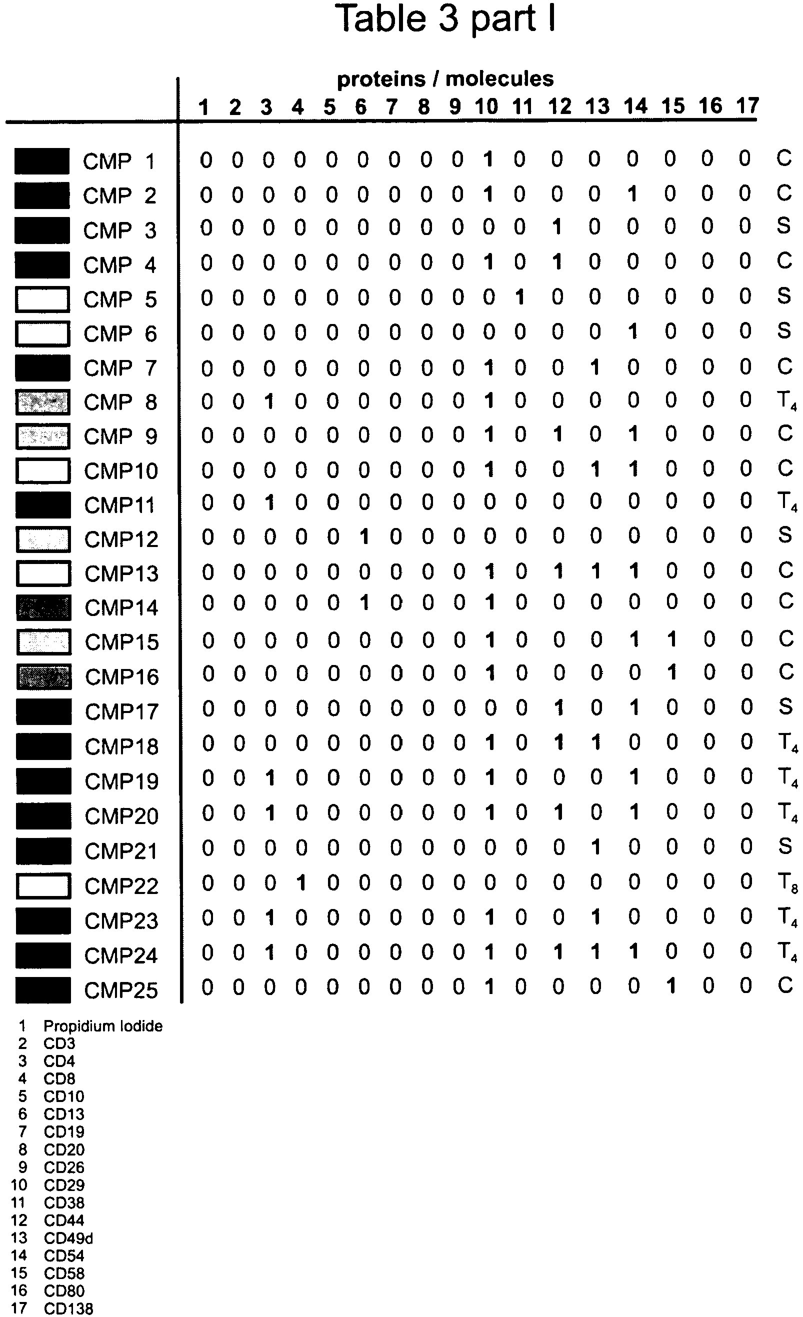

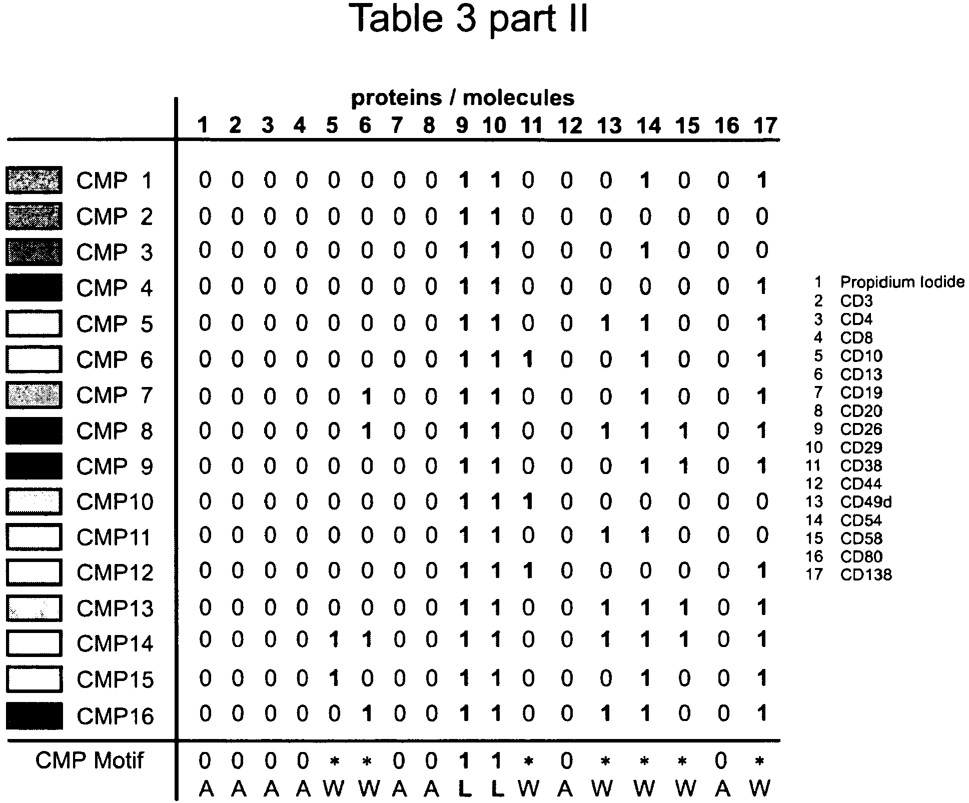

### Supplementary Table

| **CMP** | **prop** | **cd3** | **cd4** | **cd8** | **cd10** | **cd13** | **cd19** | **cd20** | **cd26** | **cd29** | **cd38** | **cd44** | **cd49d** | **cd54** | **cd58** | **cd80** | **cd138** | **freq.** |
|---|---|---|---|---|---|---|---|---|---|---|---|---|---|---|---|---|---|---|
| 1 | 0 | 0 | 0 | 0 | 0 | 0 | 0 | 0 | 0 | 1 | 0 | 0 | 0 | 0 | 0 | 0 | 0 | 24.087 |
| 2 | 0 | 0 | 0 | 0 | 0 | 0 | 0 | 0 | 1 | 1 | 0 | 0 | 0 | 1 | 0 | 0 | 1 | 6.671 |
| 3 | 0 | 0 | 0 | 0 | 0 | 0 | 0 | 0 | 0 | 1 | 0 | 0 | 0 | 1 | 0 | 0 | 0 | 5.935 |
| 4 | 0 | 0 | 0 | 0 | 0 | 0 | 0 | 0 | 0 | 0 | 0 | 1 | 0 | 0 | 0 | 0 | 0 | 5.623 |
| 5 | 0 | 0 | 0 | 0 | 0 | 0 | 0 | 0 | 1 | 1 | 0 | 0 | 0 | 0 | 0 | 0 | 0 | 5.271 |
| 6 | 0 | 0 | 0 | 0 | 0 | 0 | 0 | 0 | 1 | 0 | 0 | 0 | 0 | 0 | 0 | 0 | 0 | 4.779 |
| 7 | 0 | 0 | 0 | 0 | 0 | 0 | 0 | 0 | 1 | 1 | 0 | 0 | 0 | 1 | 0 | 0 | 0 | 4.434 |
| 8 | 0 | 0 | 0 | 0 | 0 | 0 | 0 | 0 | 0 | 1 | 0 | 1 | 0 | 0 | 0 | 0 | 0 | 4.074 |
| 9 | 0 | 0 | 0 | 0 | 0 | 0 | 0 | 0 | 0 | 0 | 1 | 0 | 0 | 0 | 0 | 0 | 0 | 3.831 |
| 10 | 0 | 0 | 0 | 0 | 0 | 0 | 0 | 0 | 0 | 0 | 0 | 0 | 0 | 1 | 0 | 0 | 0 | 3.576 |
| 11 | 0 | 0 | 0 | 0 | 0 | 0 | 0 | 0 | 0 | 1 | 0 | 0 | 1 | 0 | 0 | 0 | 0 | 3.501 |
| 12 | 0 | 0 | 1 | 0 | 0 | 0 | 0 | 0 | 0 | 1 | 0 | 0 | 0 | 0 | 0 | 0 | 0 | 2.522 |
| 13 | 0 | 0 | 0 | 0 | 0 | 0 | 0 | 0 | 0 | 1 | 0 | 1 | 0 | 1 | 0 | 0 | 0 | 2.383 |
| 14 | 0 | 0 | 0 | 0 | 0 | 0 | 0 | 0 | 1 | 1 | 0 | 0 | 0 | 0 | 0 | 0 | 1 | 1.776 |
| 15 | 0 | 0 | 0 | 0 | 0 | 0 | 0 | 0 | 1 | 0 | 0 | 0 | 0 | 1 | 0 | 0 | 0 | 1.686 |
| 16 | 0 | 0 | 0 | 0 | 0 | 0 | 0 | 0 | 0 | 1 | 0 | 0 | 1 | 1 | 0 | 0 | 0 | 1.572 |
| 17 | 0 | 0 | 0 | 0 | 0 | 0 | 0 | 0 | 0 | 1 | 1 | 0 | 0 | 0 | 0 | 0 | 0 | 1.540 |
| 18 | 0 | 0 | 1 | 0 | 0 | 0 | 0 | 0 | 0 | 0 | 0 | 0 | 0 | 0 | 0 | 0 | 0 | 1.387 |
| 19 | 0 | 0 | 0 | 0 | 0 | 1 | 0 | 0 | 0 | 0 | 0 | 0 | 0 | 0 | 0 | 0 | 0 | 1.360 |
| 20 | 0 | 0 | 0 | 0 | 0 | 0 | 0 | 0 | 1 | 1 | 0 | 0 | 1 | 1 | 0 | 0 | 1 | 1.322 |
| 21 | 0 | 0 | 0 | 0 | 0 | 0 | 0 | 0 | 1 | 0 | 0 | 0 | 0 | 1 | 0 | 0 | 1 | 1.317 |
| 22 | 0 | 0 | 0 | 0 | 0 | 0 | 0 | 0 | 0 | 1 | 0 | 1 | 1 | 1 | 0 | 0 | 0 | 1.276 |
| 23 | 0 | 0 | 0 | 0 | 0 | 1 | 0 | 0 | 0 | 1 | 0 | 0 | 0 | 0 | 0 | 0 | 0 | 1.220 |
| 24 | 0 | 0 | 0 | 0 | 0 | 0 | 0 | 0 | 1 | 1 | 1 | 0 | 0 | 1 | 0 | 0 | 1 | 1.069 |
| 25 | 0 | 0 | 0 | 0 | 0 | 0 | 0 | 0 | 0 | 0 | 1 | 1 | 0 | 0 | 0 | 0 | 0 | 1.068 |
| 26 | 0 | 0 | 0 | 0 | 1 | 0 | 0 | 0 | 1 | 0 | 0 | 0 | 0 | 0 | 0 | 0 | 0 | 1.038 |
| 27 | 0 | 0 | 0 | 0 | 0 | 0 | 0 | 0 | 1 | 0 | 0 | 0 | 0 | 0 | 0 | 0 | 1 | 955 |
| 28 | 0 | 0 | 0 | 0 | 0 | 0 | 0 | 0 | 0 | 1 | 0 | 0 | 0 | 1 | 1 | 0 | 0 | 886 |
| 29 | 0 | 0 | 0 | 0 | 0 | 0 | 0 | 0 | 0 | 1 | 0 | 0 | 0 | 0 | 1 | 0 | 0 | 875 |
| 30 | 0 | 0 | 0 | 0 | 0 | 0 | 0 | 0 | 0 | 0 | 0 | 1 | 0 | 1 | 0 | 0 | 0 | 873 |
| 31 | 0 | 0 | 0 | 0 | 0 | 0 | 0 | 0 | 0 | 1 | 0 | 1 | 1 | 0 | 0 | 0 | 0 | 865 |
| 32 | 0 | 0 | 1 | 0 | 0 | 0 | 0 | 0 | 0 | 1 | 0 | 0 | 0 | 1 | 0 | 0 | 0 | 815 |
| 33 | 0 | 0 | 0 | 0 | 0 | 1 | 0 | 0 | 1 | 1 | 0 | 0 | 0 | 1 | 0 | 0 | 1 | 806 |
| 34 | 0 | 0 | 1 | 0 | 0 | 0 | 0 | 0 | 0 | 1 | 0 | 1 | 0 | 1 | 0 | 0 | 0 | 784 |
| 35 | 0 | 0 | 0 | 0 | 0 | 1 | 0 | 0 | 1 | 1 | 0 | 0 | 1 | 1 | 1 | 0 | 1 | 761 |
| 36 | 0 | 0 | 0 | 0 | 0 | 0 | 0 | 0 | 0 | 0 | 0 | 0 | 1 | 0 | 0 | 0 | 0 | 747 |
| 37 | 0 | 0 | 0 | 0 | 0 | 0 | 0 | 0 | 1 | 1 | 0 | 0 | 0 | 1 | 1 | 0 | 1 | 726 |
| 38 | 0 | 0 | 0 | 1 | 0 | 0 | 0 | 0 | 0 | 0 | 0 | 0 | 0 | 0 | 0 | 0 | 0 | 713 |
| 39 | 0 | 0 | 0 | 0 | 0 | 0 | 0 | 0 | 1 | 1 | 1 | 0 | 0 | 0 | 0 | 0 | 0 | 697 |
| 40 | 0 | 0 | 1 | 0 | 0 | 0 | 0 | 0 | 0 | 1 | 0 | 0 | 1 | 0 | 0 | 0 | 0 | 678 |
| 41 | 0 | 0 | 1 | 0 | 0 | 0 | 0 | 0 | 0 | 1 | 0 | 1 | 1 | 1 | 0 | 0 | 0 | 665 |
| 42 | 0 | 0 | 0 | 0 | 1 | 0 | 0 | 0 | 1 | 0 | 1 | 0 | 0 | 0 | 0 | 0 | 0 | 640 |
| 43 | 0 | 0 | 0 | 0 | 0 | 0 | 0 | 0 | 0 | 0 | 0 | 0 | 0 | 0 | 1 | 0 | 0 | 617 |
| 44 | 0 | 0 | 0 | 0 | 0 | 0 | 0 | 0 | 0 | 1 | 1 | 1 | 0 | 0 | 0 | 0 | 0 | 586 |
| 45 | 0 | 0 | 0 | 0 | 0 | 0 | 0 | 0 | 1 | 0 | 1 | 0 | 0 | 0 | 0 | 0 | 0 | 581 |
| 46 | 0 | 0 | 0 | 0 | 0 | 0 | 0 | 0 | 1 | 1 | 0 | 0 | 1 | 1 | 0 | 0 | 0 | 540 |
| 47 | 0 | 0 | 0 | 0 | 0 | 0 | 0 | 0 | 1 | 1 | 1 | 0 | 0 | 0 | 0 | 0 | 1 | 527 |
| 48 | 0 | 0 | 0 | 0 | 0 | 0 | 0 | 0 | 1 | 1 | 0 | 0 | 1 | 1 | 1 | 0 | 1 | 492 |
| 49 | 0 | 0 | 1 | 0 | 0 | 0 | 0 | 0 | 0 | 0 | 0 | 1 | 0 | 0 | 0 | 0 | 0 | 461 |
| 50 | 0 | 0 | 0 | 0 | 0 | 0 | 0 | 0 | 0 | 1 | 0 | 1 | 0 | 1 | 1 | 0 | 0 | 454 |
| 51 | 0 | 0 | 0 | 0 | 1 | 1 | 0 | 0 | 1 | 1 | 0 | 0 | 1 | 1 | 1 | 0 | 1 | 451 |
| 52 | 0 | 0 | 0 | 0 | 0 | 0 | 0 | 0 | 0 | 1 | 0 | 0 | 0 | 1 | 0 | 0 | 1 | 441 |
| 53 | 0 | 0 | 0 | 0 | 1 | 0 | 0 | 0 | 1 | 1 | 0 | 0 | 0 | 1 | 0 | 0 | 1 | 428 |
| 54 | 0 | 0 | 0 | 0 | 0 | 1 | 0 | 0 | 1 | 1 | 0 | 0 | 1 | 1 | 0 | 0 | 1 | 425 |
| 55 | 0 | 0 | 0 | 0 | 0 | 0 | 0 | 0 | 0 | 0 | 0 | 0 | 0 | 1 | 1 | 0 | 0 | 403 |
| 56 | 0 | 0 | 0 | 0 | 0 | 0 | 0 | 0 | 0 | 1 | 0 | 0 | 0 | 0 | 0 | 1 | 0 | 393 |
| 57 | 0 | 0 | 0 | 0 | 0 | 1 | 0 | 0 | 0 | 1 | 0 | 0 | 0 | 1 | 0 | 0 | 0 | 385 |
| 58 | 0 | 0 | 1 | 0 | 0 | 0 | 0 | 0 | 0 | 0 | 0 | 0 | 0 | 1 | 0 | 0 | 0 | 380 |
| 59 | 0 | 0 | 1 | 0 | 0 | 0 | 0 | 0 | 0 | 1 | 0 | 0 | 1 | 1 | 0 | 0 | 0 | 375 |
| 60 | 0 | 0 | 1 | 0 | 0 | 0 | 0 | 0 | 0 | 1 | 0 | 1 | 0 | 0 | 0 | 0 | 0 | 363 |
| 61 | 0 | 0 | 0 | 0 | 0 | 1 | 0 | 0 | 1 | 1 | 0 | 0 | 0 | 1 | 1 | 0 | 1 | 358 |
| 62 | 0 | 0 | 1 | 0 | 0 | 0 | 0 | 0 | 0 | 0 | 0 | 1 | 0 | 1 | 0 | 0 | 0 | 355 |
| 63 | 0 | 0 | 0 | 0 | 0 | 0 | 0 | 0 | 0 | 1 | 0 | 0 | 0 | 0 | 0 | 0 | 1 | 352 |
| 64 | 0 | 0 | 0 | 0 | 0 | 0 | 0 | 0 | 1 | 1 | 0 | 0 | 1 | 0 | 0 | 0 | 0 | 352 |
| 65 | 0 | 0 | 0 | 0 | 0 | 0 | 0 | 0 | 1 | 1 | 1 | 0 | 0 | 1 | 0 | 0 | 0 | 342 |
| 66 | 0 | 0 | 0 | 0 | 0 | 1 | 0 | 0 | 0 | 1 | 0 | 0 | 0 | 1 | 1 | 0 | 0 | 334 |
| 67 | 0 | 0 | 0 | 0 | 0 | 1 | 0 | 0 | 0 | 1 | 0 | 0 | 0 | 0 | 1 | 0 | 0 | 334 |
| 68 | 0 | 0 | 0 | 0 | 0 | 1 | 0 | 0 | 0 | 1 | 0 | 0 | 1 | 0 | 0 | 0 | 0 | 308 |
| 69 | 0 | 0 | 1 | 0 | 0 | 0 | 0 | 0 | 0 | 1 | 1 | 1 | 0 | 1 | 0 | 0 | 0 | 307 |
| 70 | 0 | 0 | 0 | 0 | 0 | 1 | 0 | 0 | 1 | 1 | 0 | 0 | 0 | 1 | 0 | 0 | 0 | 307 |
| 71 | 0 | 0 | 0 | 0 | 0 | 0 | 0 | 0 | 1 | 0 | 1 | 0 | 0 | 0 | 0 | 0 | 1 | 307 |
| 72 | 0 | 0 | 0 | 0 | 1 | 0 | 0 | 0 | 1 | 0 | 0 | 0 | 0 | 1 | 0 | 0 | 0 | 304 |
| 73 | 0 | 0 | 0 | 0 | 0 | 0 | 0 | 0 | 1 | 1 | 0 | 1 | 0 | 1 | 0 | 0 | 0 | 302 |
| 74 | 0 | 1 | 1 | 0 | 0 | 0 | 0 | 0 | 0 | 0 | 0 | 0 | 0 | 0 | 0 | 0 | 0 | 296 |
| 75 | 0 | 0 | 0 | 0 | 1 | 0 | 0 | 0 | 1 | 1 | 0 | 0 | 1 | 1 | 0 | 0 | 1 | 284 |
| 76 | 0 | 0 | 0 | 1 | 0 | 0 | 0 | 0 | 0 | 1 | 0 | 0 | 0 | 0 | 0 | 0 | 0 | 284 |
| 77 | 0 | 1 | 0 | 0 | 0 | 0 | 0 | 0 | 0 | 0 | 0 | 0 | 0 | 0 | 0 | 0 | 0 | 280 |
| 78 | 0 | 0 | 1 | 0 | 0 | 0 | 0 | 0 | 0 | 0 | 1 | 1 | 0 | 0 | 0 | 0 | 0 | 279 |
| 79 | 0 | 0 | 1 | 1 | 1 | 1 | 0 | 0 | 1 | 0 | 0 | 0 | 1 | 0 | 0 | 0 | 0 | 278 |
| 80 | 0 | 0 | 0 | 0 | 0 | 0 | 0 | 0 | 1 | 1 | 1 | 0 | 1 | 1 | 0 | 0 | 1 | 272 |
| 81 | 0 | 0 | 1 | 0 | 0 | 0 | 0 | 0 | 0 | 1 | 1 | 1 | 1 | 1 | 0 | 0 | 0 | 270 |
| 82 | 0 | 0 | 0 | 0 | 1 | 0 | 0 | 0 | 1 | 1 | 0 | 0 | 0 | 1 | 0 | 0 | 0 | 267 |
| 83 | 0 | 0 | 0 | 0 | 0 | 1 | 0 | 0 | 0 | 0 | 0 | 0 | 0 | 0 | 1 | 0 | 0 | 253 |
| 84 | 0 | 0 | 0 | 0 | 0 | 1 | 0 | 0 | 1 | 1 | 0 | 0 | 0 | 0 | 0 | 0 | 0 | 252 |
| 85 | 0 | 0 | 1 | 0 | 0 | 0 | 0 | 0 | 0 | 0 | 1 | 1 | 0 | 1 | 0 | 0 | 0 | 248 |
| 86 | 0 | 0 | 0 | 0 | 0 | 0 | 0 | 0 | 0 | 0 | 0 | 0 | 0 | 0 | 0 | 1 | 0 | 248 |
| 87 | 0 | 0 | 0 | 0 | 0 | 0 | 0 | 0 | 0 | 1 | 1 | 0 | 1 | 0 | 0 | 0 | 0 | 236 |
| 88 | 0 | 0 | 0 | 0 | 0 | 1 | 0 | 0 | 0 | 1 | 0 | 1 | 0 | 0 | 0 | 0 | 0 | 224 |
| 89 | 0 | 0 | 0 | 0 | 1 | 1 | 0 | 0 | 1 | 1 | 0 | 0 | 1 | 1 | 0 | 0 | 1 | 223 |
| 90 | 0 | 0 | 0 | 0 | 0 | 1 | 0 | 0 | 0 | 0 | 0 | 1 | 0 | 0 | 0 | 0 | 0 | 222 |
| 91 | 0 | 0 | 0 | 0 | 0 | 0 | 0 | 0 | 1 | 1 | 0 | 1 | 0 | 0 | 0 | 0 | 0 | 216 |
| 92 | 0 | 0 | 1 | 0 | 0 | 1 | 0 | 0 | 0 | 1 | 0 | 0 | 0 | 0 | 0 | 0 | 0 | 212 |
| 93 | 0 | 0 | 0 | 0 | 0 | 0 | 0 | 0 | 0 | 0 | 0 | 0 | 0 | 0 | 0 | 0 | 1 | 212 |
| 94 | 0 | 0 | 0 | 0 | 0 | 0 | 0 | 0 | 0 | 1 | 0 | 1 | 1 | 1 | 1 | 0 | 0 | 211 |
| 95 | 0 | 0 | 0 | 0 | 0 | 0 | 0 | 0 | 0 | 1 | 0 | 0 | 1 | 1 | 1 | 0 | 0 | 208 |
| 96 | 0 | 0 | 0 | 0 | 0 | 0 | 0 | 0 | 0 | 1 | 0 | 1 | 0 | 0 | 1 | 0 | 0 | 204 |
| 97 | 0 | 0 | 0 | 0 | 0 | 0 | 0 | 0 | 0 | 0 | 0 | 1 | 1 | 0 | 0 | 0 | 0 | 201 |
| 98 | 0 | 0 | 0 | 0 | 1 | 0 | 0 | 0 | 1 | 0 | 0 | 0 | 1 | 0 | 0 | 0 | 0 | 199 |
| 99 | 0 | 0 | 0 | 0 | 0 | 0 | 0 | 0 | 0 | 0 | 0 | 0 | 0 | 1 | 0 | 0 | 1 | 199 |
| 100 | 0 | 0 | 1 | 0 | 0 | 0 | 0 | 0 | 0 | 1 | 0 | 0 | 0 | 0 | 1 | 0 | 0 | 196 |
| 101 | 0 | 1 | 1 | 0 | 0 | 0 | 0 | 0 | 0 | 0 | 0 | 1 | 0 | 0 | 0 | 0 | 0 | 195 |
| 102 | 0 | 0 | 1 | 0 | 0 | 0 | 0 | 0 | 0 | 1 | 1 | 0 | 0 | 0 | 0 | 0 | 0 | 194 |
| 103 | 0 | 0 | 0 | 0 | 0 | 0 | 0 | 0 | 0 | 1 | 0 | 0 | 1 | 0 | 1 | 0 | 0 | 188 |
| 104 | 0 | 0 | 1 | 0 | 0 | 0 | 0 | 0 | 0 | 1 | 0 | 0 | 0 | 1 | 1 | 0 | 0 | 188 |
| 105 | 0 | 1 | 1 | 0 | 0 | 0 | 0 | 0 | 0 | 1 | 0 | 0 | 0 | 0 | 0 | 0 | 0 | 186 |
| 106 | 0 | 0 | 0 | 0 | 0 | 1 | 0 | 0 | 0 | 1 | 0 | 0 | 1 | 1 | 0 | 0 | 0 | 180 |
| 107 | 0 | 0 | 0 | 0 | 0 | 1 | 0 | 0 | 1 | 0 | 0 | 0 | 0 | 1 | 0 | 0 | 1 | 178 |
| 108 | 0 | 0 | 0 | 0 | 0 | 0 | 0 | 0 | 0 | 1 | 1 | 1 | 0 | 1 | 0 | 0 | 0 | 178 |
| 109 | 0 | 0 | 1 | 0 | 0 | 0 | 0 | 0 | 0 | 1 | 0 | 1 | 0 | 1 | 1 | 0 | 0 | 174 |
| 110 | 0 | 0 | 0 | 0 | 1 | 1 | 0 | 0 | 1 | 0 | 0 | 0 | 1 | 0 | 0 | 0 | 0 | 173 |
| 111 | 0 | 0 | 0 | 0 | 0 | 1 | 0 | 0 | 0 | 1 | 0 | 1 | 0 | 1 | 0 | 0 | 0 | 172 |
| 112 | 0 | 0 | 1 | 0 | 0 | 0 | 0 | 0 | 0 | 1 | 1 | 1 | 1 | 1 | 1 | 0 | 0 | 171 |
| 113 | 0 | 0 | 0 | 1 | 0 | 0 | 0 | 0 | 0 | 0 | 0 | 1 | 0 | 0 | 0 | 0 | 0 | 170 |
| 114 | 0 | 1 | 0 | 0 | 0 | 0 | 0 | 0 | 0 | 1 | 0 | 0 | 0 | 0 | 0 | 0 | 0 | 170 |
| 115 | 0 | 0 | 0 | 0 | 0 | 1 | 0 | 0 | 0 | 1 | 0 | 0 | 1 | 1 | 1 | 0 | 0 | 167 |
| 116 | 0 | 0 | 0 | 0 | 0 | 1 | 0 | 0 | 0 | 1 | 0 | 1 | 0 | 1 | 1 | 0 | 0 | 165 |
| 117 | 0 | 0 | 1 | 0 | 0 | 0 | 0 | 0 | 0 | 0 | 1 | 0 | 0 | 0 | 0 | 0 | 0 | 164 |
| 118 | 0 | 0 | 0 | 0 | 1 | 1 | 0 | 0 | 1 | 1 | 0 | 0 | 0 | 1 | 0 | 0 | 1 | 163 |
| 119 | 0 | 0 | 0 | 0 | 0 | 0 | 0 | 0 | 0 | 0 | 0 | 1 | 0 | 0 | 1 | 0 | 0 | 162 |
| 120 | 0 | 0 | 0 | 0 | 0 | 0 | 0 | 0 | 1 | 1 | 0 | 0 | 1 | 0 | 0 | 0 | 1 | 161 |
| 121 | 0 | 1 | 0 | 0 | 0 | 0 | 0 | 0 | 0 | 0 | 0 | 1 | 0 | 0 | 0 | 0 | 0 | 160 |
| 122 | 0 | 0 | 0 | 0 | 0 | 1 | 0 | 0 | 1 | 0 | 0 | 0 | 0 | 0 | 0 | 0 | 0 | 160 |
| 123 | 0 | 0 | 1 | 0 | 0 | 0 | 0 | 0 | 0 | 1 | 0 | 1 | 1 | 1 | 1 | 0 | 0 | 159 |
| 124 | 0 | 0 | 0 | 0 | 0 | 0 | 0 | 0 | 0 | 0 | 1 | 0 | 0 | 1 | 0 | 0 | 0 | 159 |
| 125 | 0 | 0 | 0 | 0 | 0 | 1 | 0 | 0 | 0 | 0 | 0 | 0 | 0 | 1 | 0 | 0 | 0 | 159 |
| 126 | 0 | 0 | 0 | 0 | 1 | 0 | 0 | 0 | 1 | 1 | 1 | 0 | 0 | 1 | 0 | 0 | 1 | 155 |
| 127 | 0 | 0 | 0 | 0 | 0 | 0 | 0 | 0 | 1 | 1 | 0 | 0 | 0 | 1 | 1 | 0 | 0 | 154 |
| 128 | 0 | 0 | 0 | 0 | 1 | 1 | 0 | 0 | 1 | 1 | 0 | 0 | 1 | 1 | 1 | 1 | 1 | 153 |
| 129 | 0 | 0 | 0 | 0 | 0 | 0 | 0 | 0 | 0 | 1 | 1 | 0 | 0 | 0 | 0 | 0 | 1 | 149 |
| 130 | 0 | 0 | 1 | 1 | 0 | 0 | 0 | 0 | 0 | 0 | 0 | 0 | 0 | 0 | 0 | 0 | 0 | 147 |
| 131 | 0 | 1 | 1 | 0 | 0 | 0 | 0 | 0 | 0 | 1 | 0 | 1 | 0 | 1 | 0 | 0 | 0 | 144 |
| 132 | 0 | 0 | 0 | 1 | 1 | 1 | 0 | 0 | 1 | 0 | 0 | 0 | 1 | 0 | 0 | 0 | 0 | 142 |
| 133 | 0 | 0 | 0 | 0 | 1 | 0 | 0 | 0 | 1 | 0 | 0 | 0 | 0 | 1 | 0 | 0 | 1 | 139 |
| 134 | 0 | 0 | 0 | 0 | 0 | 1 | 0 | 0 | 1 | 1 | 0 | 0 | 1 | 1 | 0 | 0 | 0 | 135 |
| 135 | 0 | 0 | 0 | 0 | 0 | 1 | 0 | 0 | 1 | 0 | 0 | 0 | 0 | 1 | 0 | 0 | 0 | 135 |
| 136 | 0 | 0 | 1 | 0 | 0 | 0 | 0 | 0 | 0 | 1 | 0 | 1 | 1 | 0 | 0 | 0 | 0 | 129 |
| 137 | 0 | 0 | 1 | 0 | 0 | 1 | 0 | 0 | 0 | 1 | 1 | 1 | 1 | 1 | 1 | 0 | 0 | 128 |
| 138 | 0 | 0 | 0 | 0 | 1 | 0 | 0 | 0 | 1 | 1 | 0 | 0 | 1 | 1 | 1 | 0 | 1 | 127 |
| 139 | 0 | 0 | 0 | 0 | 1 | 1 | 0 | 0 | 1 | 1 | 0 | 0 | 0 | 1 | 1 | 0 | 1 | 124 |
| 140 | 0 | 0 | 0 | 0 | 0 | 0 | 0 | 0 | 0 | 1 | 1 | 0 | 0 | 1 | 0 | 0 | 0 | 123 |
| 141 | 0 | 0 | 0 | 0 | 0 | 1 | 0 | 0 | 1 | 1 | 0 | 0 | 0 | 0 | 0 | 0 | 1 | 123 |
| 142 | 0 | 0 | 0 | 1 | 0 | 0 | 0 | 0 | 0 | 0 | 1 | 0 | 0 | 0 | 0 | 0 | 0 | 120 |
| 143 | 0 | 0 | 0 | 0 | 0 | 1 | 0 | 0 | 0 | 1 | 0 | 1 | 1 | 1 | 0 | 0 | 0 | 120 |
| 144 | 0 | 0 | 0 | 0 | 0 | 0 | 0 | 0 | 0 | 0 | 1 | 1 | 0 | 1 | 0 | 0 | 0 | 119 |
| 145 | 0 | 0 | 0 | 0 | 0 | 0 | 0 | 0 | 0 | 1 | 0 | 0 | 1 | 1 | 0 | 0 | 1 | 117 |
| 146 | 0 | 0 | 0 | 0 | 0 | 0 | 0 | 0 | 1 | 0 | 0 | 0 | 1 | 1 | 0 | 0 | 1 | 117 |
| 147 | 0 | 1 | 1 | 0 | 0 | 0 | 0 | 0 | 0 | 1 | 0 | 0 | 0 | 1 | 0 | 0 | 0 | 116 |
| 148 | 0 | 0 | 0 | 0 | 0 | 0 | 0 | 0 | 0 | 1 | 1 | 1 | 1 | 0 | 0 | 0 | 0 | 114 |
| 149 | 0 | 0 | 0 | 0 | 0 | 1 | 0 | 0 | 0 | 0 | 0 | 0 | 0 | 1 | 1 | 0 | 0 | 114 |
| 150 | 0 | 1 | 1 | 0 | 0 | 0 | 0 | 0 | 0 | 1 | 0 | 1 | 0 | 0 | 0 | 0 | 0 | 113 |
| 151 | 0 | 0 | 0 | 0 | 0 | 1 | 0 | 0 | 0 | 1 | 0 | 1 | 1 | 1 | 1 | 0 | 0 | 113 |
| 152 | 0 | 0 | 1 | 0 | 0 | 0 | 0 | 0 | 0 | 1 | 1 | 1 | 0 | 1 | 1 | 0 | 0 | 112 |
| 153 | 0 | 0 | 0 | 0 | 0 | 0 | 0 | 0 | 0 | 0 | 0 | 1 | 0 | 1 | 1 | 0 | 0 | 110 |
| 154 | 0 | 1 | 1 | 0 | 0 | 0 | 0 | 0 | 0 | 1 | 0 | 1 | 1 | 1 | 0 | 0 | 0 | 109 |
| 155 | 0 | 0 | 0 | 0 | 0 | 1 | 0 | 0 | 1 | 1 | 1 | 0 | 0 | 1 | 0 | 0 | 1 | 107 |
| 156 | 0 | 1 | 1 | 0 | 0 | 0 | 0 | 0 | 0 | 1 | 1 | 1 | 0 | 1 | 0 | 0 | 0 | 107 |
| 157 | 0 | 0 | 0 | 0 | 0 | 1 | 0 | 0 | 0 | 1 | 0 | 0 | 1 | 0 | 1 | 0 | 0 | 106 |
| 158 | 0 | 0 | 0 | 0 | 0 | 0 | 0 | 0 | 1 | 1 | 0 | 1 | 1 | 1 | 0 | 0 | 0 | 102 |
| 159 | 0 | 0 | 1 | 0 | 0 | 1 | 0 | 0 | 0 | 0 | 0 | 0 | 0 | 0 | 0 | 0 | 0 | 101 |
| 160 | 0 | 0 | 0 | 0 | 0 | 1 | 0 | 0 | 1 | 0 | 0 | 0 | 0 | 0 | 0 | 0 | 1 | 101 |
| 161 | 0 | 0 | 0 | 0 | 1 | 1 | 0 | 0 | 1 | 0 | 0 | 0 | 1 | 1 | 1 | 0 | 0 | 101 |
| 162 | 0 | 0 | 0 | 0 | 1 | 0 | 0 | 0 | 1 | 1 | 0 | 0 | 0 | 1 | 1 | 0 | 1 | 100 |
| 163 | 0 | 0 | 0 | 0 | 0 | 0 | 0 | 0 | 0 | 1 | 0 | 0 | 0 | 0 | 1 | 1 | 0 | 100 |
| 164 | 0 | 0 | 0 | 0 | 0 | 0 | 0 | 0 | 0 | 0 | 0 | 0 | 1 | 1 | 0 | 0 | 0 | 100 |
| 165 | 0 | 1 | 1 | 0 | 0 | 0 | 0 | 0 | 0 | 1 | 1 | 1 | 1 | 1 | 0 | 0 | 0 | 100 |

## Claims

1. A method for identifying prostate cancer in a patient comprising at least the step of examining if at least a protein cluster (CMP) or a group of CMPs containing at least CD26 and CD29 can be identified on the cell surfaces of cells from a part of the body of the patient, wherein said part of the body is prostate tissue and is suspected to be affected by prostate cancer.

2. The method according to claim 1 wherein at least one protein cluster (CMP) or group of CMPs also containing CD44 and/or CD54 and/or CD138 and lacking at least one of CD3, CD4, CD8, CD10, CD13, CD19, CD20, CD38, CD49d, CD58, and CD80 is identified on the cell surfaces of said cells.

3. The method according to claim 1 wherein at least one protein cluster (CMP) or group of CMPs lacking at least one of CD3, CD4, CD8, CD10, CD13, CD19, CD20, CD38, CD49d, CD58, CD80, CD44, CD54, and/or CD138 is identified on the cell surfaces of said cells.

4. The method according to one of claims 1 to 3 wherein the disease is identified as prostate cancer if the cells derive from stroma and/or neoplastic epithelium of acini of prostate tissue and/or if at least 70%, preferably at least 75%, of the CMPs on the cell surface of at least one cell contain CD26 and/or CD29 and/or if at least 20%, preferably at least 35%, of the CMPs on the cell surface of at least one cell comprise CD26 and/or CD29 and lack at least CD3, CD4, CD8, CD10, CD13, CD19, CD20, CD38, CD49d, CD58, and CD80.

## Patentansprüche

1. Verfahren zum Identifizieren von Prostatakrebs bei einem Patienten mit zumindest dem Schritt des Untersuchens, ob mindestens ein Proteincluster (CMP) oder eine Gruppe von CMPs, die mindestens CD26 und CD29 enthalten, auf den Zellenoberflächen von Zellen von einem Teil des Körpers des Patienten identifiziert werden kann, wobei der Teil des Körpers Prostatagewebe ist und mutmaßlich von Prostatakrebs betroffen ist.

2. Verfahren nach Anspruch 1, wobei mindestens ein Proteincluster (CMP) oder eine Gruppe von CMPs, die auch CD44 und/oder CD54 und/oder CD138 enthalten und denen mindestens eines von CD3, CD4, CD8, CD10, CD13, CD19, CD20, CD38, CD49d, CD58 und CD80 fehlt, auf den Zellenoberflächen der Zellen identifiziert wird.

3. Verfahren nach Anspruch 1, wobei mindestens ein Proteincluster (CMP) oder eine Gruppe von CMPs, denen mindestens eines von CD3, CD4, CD8, CD10, CD13, CD19, CD20, CD38, CD49d, CD58, CD80, CD44, CD54 und/oder CD138 fehlt, auf den Zellenoberflächen der Zellen identifiziert wird.

4. Verfahren nach einem der Ansprüche 1 bis 3, wobei die Erkrankung als Prostatakrebs identifiziert wird, wenn die Zellen vom Stroma und/oder neoplastischen Epithel von Azini von Prostatagewebe stammen, und/oder wenn mindestens 70 %, vorzugsweise mindestens 75 % der CMPs auf der Zellenoberfläche mindestens einer Zelle CD26 und/oder CD29 enthalten, und/oder wenn mindestens 20 %, vorzugsweise mindestens 35 % der CMPs auf der Zellenoberfläche mindestens einer Zelle CD26 und/oder CD29 umfassen und ihnen mindestens CD3, CD4, CD8, CD10, CD13, CD19, CD20, CD38, CD49d, CD58 und CD80 fehlt.

## Revendications

1. Procédé d'identification d'un cancer de la prostate chez un patient, comprenant au moins l'étape consistant à vérifier si au moins un agrégat de protéines (CMP) ou un groupe de CMP, contenant au moins du CD26 et du CD29, peut être identifié à la surface cellulaire de cellules d'une partie du corps du patient, dans lequel ladite partie du corps est le tissu prostatique et est soupçonnée d'être affectée par un cancer de la prostate.

2. Procédé selon la revendication 1, dans lequel au moins un agrégat de protéines (CMP) ou un groupe de CMP, contenant aussi du CD44 et / ou du CD54 et / ou du CD138 et manquant au moins d'un élément parmi le CD3, le CD4, le CD8, le CD10, le CD13, le CD19, le CD20, le CD38, le CD49d, le CD58 et le CD80, est identifié à la surface cellulaire desdites cellules.

3. Procédé selon la revendication 1, dans lequel au moins un agrégat de protéines (CMP) ou un groupe de CMP, manquant au moins d'un élément parmi le CD3, le CD4, le CD8, le CD10, le CD13, le CD19, le CD20, le CD38, le CD49d, le CD58, le CD80, le CD44, le CD54 et / ou le CD138, est identifié à la surface cellulaire desdites cellules.

4. Procédé selon l'une des revendications 1 à 3, dans lequel la maladie est identifiée comme étant un cancer de la prostate si les cellules proviennent d'un stroma et / ou d'un épithélium néoplasique d'acini de tissu prostatique et / ou si au moins 70 %, de préférence au moins 75 %, des CMP à la surface cellulaire d'au moins une cellule contiennent du CD26 et / ou du CD29 et / ou si au moins 20 %, de préférence au moins 35 %, des CMP à la surface cellulaire d'au moins une cellule comprennent du CD26 et / ou du CD29 et manquent au moins de CD3, de CD4, de CD8, de CD10, de CD13, de CD19, de CD20, de CD38, de CD49d, de CD58 et de CD80.
